(19)

Europäisches
Patentamt

European
Patent Office

Office européen
des brevets

(11) **EP 4 365 298 A1**

(12) **EUROPEAN PATENT APPLICATION**
published in accordance with Art. 153(4) EPC

(43) Date of publication:
**08.05.2024 Bulletin 2024/19**

(21) Application number: **22833215.1**

(22) Date of filing: **29.06.2022**

(51) International Patent Classification (IPC):
*C12N 15/86* (2006.01)      *C12N 5/071* (2010.01)
*C12N 7/01* (2006.01)       *C12N 15/38* (2006.01)
*C12N 15/39* (2006.01)      *C12N 15/44* (2006.01)
*C12N 15/49* (2006.01)

(52) Cooperative Patent Classification (CPC):
**C07K 14/03; C07K 14/065; C07K 14/11;
C07K 14/155; C12N 5/06; C12N 7/00; C12N 15/86;
Y02A 50/30**

(86) International application number:
**PCT/JP2022/025983**

(87) International publication number:
**WO 2023/277069 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.06.2021   JP 2021108757**

(71) Applicant: **Repli-Tech Co., Ltd.
Tokyo 150-0012 (JP)**

(72) Inventor: **SAEKI, Koichi
Tokyo 150-0012 (JP)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **METHOD OF PRODUCING NEGATIVE-STRAND RNA VIRUS VECTOR AND PRODUCED
NEGATIVE-STRAND RNA VIRUS VECTOR**

(57)      The present disclosure provides a method of producing a negative-strand RNA virus vector. The present disclosure specifically provides a method of producing a negative-strand RNA virus vector in the presence of a PKR inhibitory factor.

**EP 4 365 298 A1**

## Description

### Technical Field

The present invention relates to a method of producing a negative-strand RNA virus vector and the produced negative-strand RNA virus vector.

### Background Art

Negative-strand RNA virus vectors typified by a Sendai virus vector can be useful for gene transfer or cytokine induction. The negative-strand RNA virus vectors typified by a Sendai virus vector can be advantageously produced in a packaging cell by producing the virus vector from cDNA (Patent Literature 1).

E3L of vaccinia virus is a double-stranded RNA-binding protein, and has been reported to promote interferon resistance and intracellular proliferation (Non Patent Literature 1). It is disclosed that in the production of a poxvirus or vaccinia virus, a protein kinase R (PKR) is inhibited with K3L, E3L, VAI RNA, EBER, $\sigma$3 or TRBP or a combination thereof and that the PKR is a double-stranded RNA binding protein (Patent Literature 2). It is disclosed that C8L and K3L are pseudosubstrates of a protein kinase R (PKR) (Non Patent Literature 2). It is suggested that NS5A or NS5A (1-148) may inhibit antiviral activity against HCV (Non Patent Literature 3). A method of producing a virus with VAI RNA is disclosed (Patent Literature 3).

### Citation List

### Patent Literature

Patent Literature 1: International Publication No. WO 2005/071092
Patent Literature 2: International Publication No. WO 98/040500
Patent Literature 3: International Publication No. WO 95/011983

### Non Patent Literature

Non Patent Literature 1: T. Shors, et al., Virology, 239: 269 - 276, 1997
Non Patent Literature 2: M. K. Kobayashi et al., Virology, 276 :424 - 434, 2000
Non Patent Literature 3: T. Taguchi et al., J. Gen. Virol., 85: 959 - 969, 2004

### Summary of Invention

The present invention provides to a method of producing a negative-strand RNA virus vector and the produced negative-strand RNA virus vector.

The present inventors have found that in a method of producing a negative-strand RNA virus, causing a packaging cell to express a genomic RNA of the negative-strand RNA virus to form the negative-strand RNA virus or virus vector in the presence of a PKR inhibitory factor (such as a PKR inhibitory viral factor, human nc886, human p58$^{IPK}$ or NS5A or a combination thereof) leads to an increase in the virus production level in the packaging cell.

According to the present invention, for example, the following inventions can be provided.

(1) A method of producing a negative-strand RNA virus or virus vector, the method comprising:

expressing a protein kinase R (PKR) inhibitory factor (such as a PKR inhibitory viral factor, human nc886, human p58$^{IPK}$ or NS5A, or a combination thereof) from a gene encoding the PKR inhibitory factor operably linked to a regulatory sequence and supplying it to a packaging cell;
causing the packaging cell to express a genomic RNA of the negative-strand RNA virus or virus vector to form the negative-strand RNA virus or virus vector in the presence of the PKR inhibitory factor {for example, causing the packaging cell to express a genomic RNA of the negative-strand RNA virus or virus vector to form the negative-strand RNA virus or virus vector in the presence of the PKR inhibitory factor such as a PKR inhibitory viral factor wherein the genomic RNA and the PKR inhibitory factor can be preferably expressed with the DNA according to (13) below}; and
recovering the formed negative-strand RNA virus or virus vector;
wherein preferably, the relationship between the virus or virus vector and the PKR inhibitory factor is heterologous, and/or the relationship between the regulatory sequence and the PKR inhibitory factor is heterologous.

(2) The method according to (1) above, wherein the negative-strand RNA virus or virus vector is a Sendai virus vector.
(3) The method according to (1) or (2) above, wherein the PKR inhibitory factor is one or more selected from the group consisting of VAI RNA of adenovirus, EBER of EB virus, human nc886, TAR of HIV virus, 2A$^{pro}$ of poliovirus, E3L of vaccinia virus, $\delta$3 of reovirus, NS1 of influenza virus, human p58$^{IPK}$, NS5A of hepatitis C virus, K3L of vaccinia virus, Tat of HIV virus, Us11 of herpes simplex virus and ICP34.5 of herpes simplex virus, and orthologs thereof.
(4) The method according to any of (1) to (3) above, wherein the negative-strand RNA virus or virus vector is produced in the absence of a helper virus.

(5) The method according to any of (1) to (4) above, wherein the genomic RNA further has the gene encoding the PKR inhibitory factor operably linked to the regulatory sequence.

(6) The method according to any of (1) to (5) above, wherein the packaging cell has a genomic DNA having the gene encoding the PKR inhibitory factor operably linked to the regulatory sequence.

(7) The method according to any of (1) to (6) above, wherein the packaging cell is a Vero cell or an LLC-MK2 cell.

(8) The method according to any of (1) to (7) above, wherein the packaging cell is a cell population consisting of Vero cells or a cell population consisting of LLC-MK2 cells, and does not comprise other cells.

(9) An RNA genome of a negative-strand RNA virus or virus vector, comprising expressibly a gene encoding any one or more of PKR inhibitory factors.

(10) A negative-strand RNA virus or virus vector comprising the RNA genome according to (9) above.

(11) The negative-strand RNA virus or virus vector according to (10) above, further comprising a gene of interest.

(12) A composition comprising the negative-strand RNA virus or virus vector according to (10) or (11) above.

(13) A DNA encoding the RNA genome according to (9) above.

(14) A gene expression vector comprising the DNA according to (13) above operably linked to a regulatory sequence.

(15) The composition according to (12) above, wherein the infectious titer is $1 \times 10^5$ CIU/mL or more.

(16) The composition according to (12) above, wherein the infectious titer is $1 \times 10^6$ CIU/mL or more.

(17) The composition according to (12) above, wherein the infectious titer is $1 \times 10^7$ CIU/mL or more.

(18) The method according to any of (1) to (8) above, wherein the PKR inhibitory factor has a sequence set forth in any of SEQ ID NOs: 4 to 31.

(19) The negative-strand RNA virus or virus vector according to any of (9) to (11) above, wherein the PKR inhibitory factor has a sequence set forth in any of SEQ ID NOs: 4 to 31.

(20) The composition according to (12) above, wherein the PKR inhibitory factor has a sequence set forth in any of SEQ ID NOs: 4 to 31.

(21) The DNA according to (13) above, wherein the PKR inhibitory factor has a sequence set forth in any of SEQ ID NOs: 4 to 31.

(22) The gene expression vector according to (14) above, wherein the PKR inhibitory factor has a sequence set forth in any of SEQ ID NOs: 4 to 31.

(23) The method according to any of (1) to (8) above, wherein expressing the PKR inhibitory factor is performed by introducing, into the packaging cell, a plasmid vector having a gene encoding the PKR inhibitory factor operably linked to a first regulatory sequence.

(24) The method according to any of (1) to (8) above, wherein expressing the genomic RNA is performed by introducing, into the packaging cell, a plasmid vector having a gene encoding the genomic RNA operably linked to a second regulatory sequence.

(25) The method according to any of (1) to (8) above, wherein:

expressing the PKR inhibitory factor is performed by introducing, into the packaging cell, a plasmid vector having a gene encoding the PKR inhibitory factor operably linked to a first regulatory sequence; and
expressing the genomic RNA is performed by introducing, into the packaging cell, a plasmid vector having a gene encoding the genomic RNA operably linked to a second regulatory sequence.

(26) The method according to any of (1) to (8) above, wherein forming the negative-strand RNA virus or virus vector comprises introducing, into the packaging cell, a plasmid vector having a gene encoding a genomic RNA of a virus operably linked to a second regulatory sequence and a plasmid vector having a gene encoding a component of the virus operably linked to a third regulatory sequence; and expressing the genomic RNA and the component of the virus in the cell.

(27) The method according to (26) above, wherein the third regulatory sequence is an EF1α promoter and the component of the virus comprises either or both of N protein and L protein.

(28) The method according to (26) above, wherein the third regulatory sequence is an EF1α promoter, and the component of the virus is one or more or all selected from the group consisting of N protein, P protein, and L protein.

(29) The method according to (28) above, wherein the packaging cell is a Vero cell.

(30) The method according to (26) above, wherein the third regulatory sequence is an EF1α promoter and the component of the virus comprises either or both of P protein and L protein.

(31) The method according to (26) above, wherein the third regulatory sequence is an EF1α promoter and the component of the virus is L protein.

(32) The method according to (30) above, wherein the packaging cell is an LLC-MK2 cell.

(33) The method according to (31) above, wherein the packaging cell is an LLC-MK2 cell.

(34) The method according to any of the above, wherein the PKR inhibitory factor comprises:

(i) E3L or part thereof, preferably a peptide comprising at least C-terminal 107 amino acids of E3L; and
(ii) K3L.

(35) The method according to any of the above, wherein the PKR inhibitory factor comprises:

(i) E3L or part thereof, preferably a peptide comprising at least C-terminal 107 amino acids of E3L; and
(iii) Y3.

(36) The method according to (34) above, wherein the PKR inhibitory factor further comprises VAI.
(37) The method according to (35) above, wherein the PKR inhibitory factor further comprises VAI.
(38) The method according to any of the above, wherein the PKR inhibitory factor comprises VAI, and VAI is a molecule having a sequence set forth in SEQ ID NO: 17.
(39) The method according to any of the above, wherein the PKR inhibitory factor comprises VAI, and the PKR inhibitory factor is a molecule having a sequence set forth in SEQ ID NO: 19.
(40) The method according to any of the above, wherein the PKR inhibitory factor is a molecule having a sequence set forth in any of SEQ ID NOs: 18 and 21 to 26.
(41) The method according to any of the above, wherein the PKR inhibitory factor comprises nc886.
(42) The method according to any of the above, wherein the PKR inhibitory factor is a molecule having a sequence set forth in SEQ ID NO: 13 or 14.
(43) The method according to (5) above, wherein the genomic RNA has a gene encoding a protein of interest; the PKR inhibitory factor is VAI or an RNA molecule having a sequence set forth in any of SEQ ID NOs: 17 to 26; and VAI is comprised in the 3'UTR of the gene encoding the protein of interest.
(44) The method according to (43) above, wherein the packaging cell has a genomic DNA having a gene encoding the PKR inhibitory factor operably linked to a regulatory sequence.
(45) The method according to (44) or (45) above, wherein the packaging cell is a Vero cell or LLC-MK2 cell.
(46) The method according to any of (44) to (46) above, wherein the packaging cell is a cell population consisting of Vero cells or a cell population consisting of LLC-MK2 cells, and does not comprise other cells.
(47) The RNA genome according to (9) above, wherein the RNA genome has a gene encoding a protein of interest; the PKR inhibitory factor is VAI or an RNA molecule having a sequence set forth in any of SEQ ID NOs: 17 to 26; and the VAI or the RNA molecule having a sequence set forth in any of SEQ ID NOs: 17 to 26 is comprised in the 3'UTR of the gene encoding the protein of interest.
(48) A negative-strand RNA virus or virus vector comprising the RNA genome according to (47) above.
(49) A method of producing a negative-strand RNA virus or virus vector, the method comprising:

expressing the PKR inhibitory factor NS5A from a gene encoding the NS5A operably linked to a regulatory sequence and supplying it to a packaging cell;
causing the packaging cell to express a genomic RNA of the negative-strand RNA virus or virus vector to form the negative-strand RNA virus or virus vector in the presence of the NS5A; and
recovering the formed negative-strand RNA virus or virus vector;
wherein the relationship between the virus or virus vector and the NS5A is heterologous, and/or the relationship between the regulatory sequence and the NS5A is heterologous.

(50) A method of producing a negative-strand RNA virus or virus vector, the method comprising:

expressing a PKR inhibitory factor from a gene encoding the PKR inhibitory factor operably linked to a regulatory sequence and supplying it to a packaging cell, the PKR inhibitory factor being either or both of human nc886 (VTRNA2-1) and human p58$^{IPK}$;
causing the packaging cell to express a genomic RNA of the negative-strand RNA virus or virus vector to form the negative-strand RNA virus or virus vector in the presence of the PKR inhibitory factor; and
recovering the formed negative-strand RNA virus or virus vector;
wherein the relationship between the regulatory sequence and the PKR inhibitory factor is optionally heterologous.

(51) The method according to (49) or (50) above, wherein the negative-strand RNA virus or virus vector is a Sendai virus vector.
(52) The method according to any of (49) to (51) above, wherein the genomic RNA further has the gene encoding the PKR inhibitory factor operably linked to the regulatory sequence.
(53) The method according to any of (49) to (52) above, wherein the packaging cell has a genomic DNA having the gene encoding the PKR inhibitory factor operably linked to the regulatory sequence.
(54) The method according to any of (49) to (53) above, wherein the packaging cell is a Vero cell or an LLC-MK2 cell.
(55) The method according to any of (49) to (54) above, wherein the packaging cell is a cell population consisting of Vero cells or a cell population consisting of LLC-MK2 cells, and does not comprise other cells.

(56) An RNA genome of a negative-strand RNA virus or virus vector, comprising expressibly a gene encoding any one or more of PKR inhibitory factors.

(57) A negative-strand RNA virus or virus vector comprising the RNA genome according to (56) above.

(58) The negative-strand RNA virus or virus vector according to (57) above, further comprising a gene of interest.

(59) A composition comprising the negative-strand RNA virus or virus vector according to (57) or (58) above.

(60) A DNA encoding the RNA genome according to (56) above.

(61) A gene expression vector comprising the DNA according to (60) above operably linked to a regulatory sequence.

(71) A gene expression vector (preferably a plasmid) for an RNA genome of an RNA virus comprising a regulatory sequence (preferably a promoter sequence), a first DNA and a second DNA in this order, wherein:

the first DNA encodes the RNA genome of the RNA virus;
the second DNA encodes a protein kinase R (PKR) inhibitory factor (such as a PKR inhibitory viral factor (preferably VAI RNA, EBER, nc886 and TAR, and orthologs thereof)) ;
wherein the first DNA and the second DNA form a region comprising the first DNA and the second DNA in series is formed in the gene expression vector, the region being operably linked to the regulatory sequence, whereby the first DNA and the second DNA are transcribed into one RNA.

(72) The gene expression vector according to (71) above, wherein the protein kinase R (PKR) inhibitory factor is VAI.

(73) The gene expression vector according to (71) above, further comprising a self-cleaving ribozyme sequence between the first DNA and the second DNA.

(74) The gene expression vector according to (72) above, further comprising a self-cleaving ribozyme sequence between the first DNA and the second DNA.

(75) The gene expression vector according to (71) above, further comprising a self-cleaving ribozyme sequence between the regulatory sequence and the first DNA.

(76) The gene expression vector according to (72) above, further comprising a self-cleaving ribozyme sequence between the regulatory sequence and the first DNA.

(77) The gene expression vector according to (73) above, further comprising a self-cleaving ribozyme sequence between the regulatory sequence and the first DNA.

(78) The gene expression vector according to (74) above, further comprising a self-cleaving ribozyme sequence between the regulatory sequence and the first DNA.

(79) The invention according to any of the above, wherein the VAI RNA is part of SEQ ID NOs: 19, 20 and 23 to 26 and optionally has a sequence comprising a nucleotide sequence set forth in SEQ ID NO: 17.

(80) The invention according to any of the above, wherein the VAI RNA is part of SEQ ID NOs: 19, 20 and 23 to 26, and has a sequence comprising either or both of (i) the VAI RNA, which corresponds to a sequence comprising a nucleotide sequence set forth in SEQ ID NO: 17, and (ii) either or both of sequences on the 5'-side and 3'-side of the VAI RNA.

Brief Description of Drawings

[Figure 1A] Figure 1A shows the secondary structure of VAI RNA. The 74th base is indicated by an arrow.
[Figure 1B] Figure 1B shows a sequence containing VAI RNA and mutated sequences thereof.
[Figure 2] Figure 2 shows the results of an experiment in which the reconstitution efficiencies of the negative-strand RNA virus vectors produced in the presence or absence of VAI were confirmed.
[Figure 3] Figure 3 shows the results of an experiment in which the infectious titers of the negative-strand RNA virus vectors produced in Figure 2 were confirmed.
[Figure 4] Figure 4 shows the results of an experiment in which the reconstitution efficiencies of the negative-strand RNA virus vectors produced in the presence or absence of nc886 were confirmed.
[Figure 5] Figure 5 shows the results of an experiment in which the reconstitution efficiencies of the negative-strand RNA virus vectors produced in the presence or absence of PKR inhibitory viral factors were confirmed.
[Figure 6] Figure 6 shows the results of an experiment in which the infectious titers of the negative-strand RNA virus vectors produced in Figure 5 were confirmed.
[Figure 7] Figure 7 shows the results of an experiment in which the reconstitution efficiencies of the negative-strand RNA virus vectors produced in the presence or absence of fusion sequence of PKR inhibitory factors were confirmed.

[Figure 8] Figure 8 shows the results of an experiment in which expression promoters were compared in an LLC-MK2-F cell and the reconstitution efficiencies of the negative-strand RNA virus vectors produced in the presence or absence of PKR inhibitory factors were confirmed.

[Figure 9] Figure 9 shows the results of an experiment in which expression promoters were compared in a Vero-F cell and the reconstitution efficiencies of the negative-strand RNA virus vectors produced in the presence or absence of PKR inhibitory factors were confirmed.

[Figure 10] Figure 10 shows the results of an experiment in which the reconstitution efficiencies of the negative-strand RNA virus vectors produced in the presence or absence of a plurality of PKR inhibitory factors were confirmed.

[Figure 11] Figure 11 shows the results of an experiment in which the infectious titers of the negative-strand RNA virus vectors produced in Figure 10 were confirmed.

[Figure 12] Figure 12 shows the results of an experiment in which the reconstitution efficiencies of produced negative-strand RNA virus vectors having a virus genome with or without a PKR inhibitory factor were confirmed.

[Figure 13] Figure 13 shows the results of an experiment in which the infectious titers of the negative-strand RNA virus vectors produced in Figure 12 were confirmed.

[Figure 14] Figure 14 shows the results of confirming the reconstitution efficiencies of the negative-strand RNA virus vectors by VAI RNA extended to the 5' and 3' sides.

[Figure 15] Figure 15 shows the introduction of hammerhead ribozyme between the T7 promoter and an SeV genome-encoding DNA and the effect thereof on the SeV reconstitution efficiency using a packaging cell that constitutively expresses a PKR inhibitory factor.

[Figure 16] Figure 16 shows the effects of the introduction of hammerhead ribozyme between the T7 promoter and an SeV genome-encoding DNA and the introduction of HDV ribozyme between the SeV genome-encoding DNA and VAI RNA on the SeV reconstitution efficiency.

[Figure 17] Figure 17 shows the result of a PCR experiment showing that HDV ribozyme self-cleaves VAI RNA from an SeV genome.

[Figure 18] Figure 18 shows the effect of the introduction of VAI (330 bp) 74c into a plasmid for SeV reconstitution on the SeV reconstitution efficiency.

[Figure 19] Figure 19 shows the effect of the introduction of VAI (330 bp) 74c into a plasmid for SeV reconstitution and the combined use of E3Y3 therewith on the SeV reconstitution efficiency.

[Figure 20] Figure 20 shows the effect of the introduction of VAI (330 bp) 74c into a plasmid for SeV reconstitution and the combined use of E3Y3 therewith on the infectious titer of SeV.

[Figure 21A] Figure 21A shows maps of pCAG-SeV and pEF1-SeV constructs used in the experiment.

[Figure 21B] Figure 21B shows the reconstitution efficiencies of pCAG-SeV and pEF1-SeV.

[Figure 22] Figure 22 shows the effect of VAI RNA on the reconstitution efficiency of mumps virus (MuV; Paramyxoviridae Rubulavirus).

[Figure 23] Figure 23 shows the effect of VAI RNA on the reconstitution efficiency of measles virus (MeV; Paramyxoviridae Morbillivirus).

[Figure 24] Figure 24 shows the effect of E3Y3 on the reconstitution efficiency of vesicular stomatitis virus (VSV; Rhabdoviridae Vesiculovirus).

[Figure 25] Figure 25 shows the reconstitution efficiencies of SeV, MeV, and MuV using heterologous RNA polymerases.

[Figure 26] Figure 26 shows the reconstitution efficiency of VSV using a heterologous RNA polymerase.

[Figure 27A] Figure 27A shows a map of the p3vLPNP construct used in the experiment.

[Figure 27B] Figure 27B shows the result of the reconstitution of SeV using pCAG-SeV or pEF1-SeV and p3vLPNP.

Description of Embodiments

As used herein, the "negative-strand RNA virus vector" refers to a recombinant virus obtained by modifying a virus (that is, a negative-strand RNA virus) that has a negative-strand RNA as its genome for transferring a gene of interest. Examples of the negative-strand RNA virus include the family Orthomyxoviridae (orthomyxoviruses such as influenza virus), the family Paramyxoviridae (paramyxoviruses such as the genus Morbillivirus), the family Rhabdoviridae (rhabdoviruses such as rabies virus), the family Filoviridae (filoviruses such as Ebola virus and Marburg virus) and the family Bunyaviridae (bunyaviruses such as hantavirus). Examples of the paramyxovirus include viruses of the subfamily Orthoparamyxovirinae. Examples of the viruses of the subfamily Orthoparamyxovirinae include viruses of the genus Respirovirus such as a Sendai virus.

As used herein, the "protein kinase R" (PKR) is a protein-phosphorylating enzyme activated by a double-stranded RNA. The PKR is encoded by an EIF2AK2 gene in human. The PKR contains an N-terminal double-stranded RNA binding domain and a C-terminal kinase domain. The kinase domains have an apoptosis-inducing function. The PKR dimerizes by binding to the double-stranded RNA and subsequently autophosphorylates to be activated. The activated PKR phosphorylates a eukaryotic translation initiation factor eIF2$\alpha$. The phosphorylation of eIF2$\alpha$ inhibits mRNA translation in cells. The activated PKR can also induce apoptosis in cells to prevent viral spread. Some viruses have a factor that counteracts the PKR (that is, a PKR inhibitory viral factor). For example, certain viruses produce a decoy RNA that binds to the PKR to prevent its activation. Examples of the decoy RNA include VAI RNA of adenovirus (for example, having a sequence set forth in SEQ ID NO: 17), EBER of EB virus (for example, having a sequence set forth in SEQ ID NO: 4), and TAR of HIV (for example, having a sequence set forth in SEQ ID NO: 5). 2A$^{pro}$ of a poliovirus (for example, having a sequence set forth in SEQ ID NO: 6) is known as a molecule that induces PKR degradation. For the RNA-based factor, a terminator (such as a T7 terminator for a T7 polymerase) may be linked to the 3'-terminal of the nucleotide encoding the RNA. Examples of a factor that masks a double-stranded RNA of a virus to prevent activation of a PKR include E3L of vaccinia virus (for example, having a sequence set forth in SEQ ID NO: 7), $\sigma$3 of reovirus (for example, having a sequence set forth in SEQ ID NO: 8), Us11 of herpes simplex virus (for example, having a sequence set forth in SEQ ID NO: 29), and NS1 of influenza virus (for example, having a sequence set forth in SEQ ID NO: 28). Examples of pseudosubstrates include K3L of vaccinia virus (for example, having a sequence set forth in SEQ ID NO: 10) and Tat of HIV (for example, having a sequence set forth in SEQ ID NO: 11). Examples of a molecule that induces dephosphorylation of a substrate include ICP34.5 of herpes simplex virus (for example, having a sequence set forth in SEQ ID NO: 12). These factors are PKR inhibitory viral factors. These factors may be of natural form.

As used herein, the "nc886" is a non-coding RNA, also referred to as VTRNA2-1, CBL3, and hvg-5. The nc886 functions as a direct inhibitor of PKR. The nc886 can have, for example, a sequence set forth in SEQ ID NO: 13. As used herein, the "p58$^{IPK}$" is a protein, found as a cytoplasmic protein, which functions as an inhibitor of PKR. The p58$^{IPK}$ can be, for example, human p58$^{IPK}$, and may have, for example, a sequence set forth in SEQ ID NO: 9. These factors may be of natural form.

As used herein, the "NS5A" is a nonstructural protein possessed by hepatitis C virus (HCV). The NS5A is a phosphorylated protein and is considered to be essential for HCV genome replication. Furthermore, the NS5A (for example, having a sequence set forth in SEQ ID NO: 15) can inhibit antiviral activities against HCV and encephalomyocarditis virus (Medical Journal of Kobe University, 2003, 64 (1/2): 7 - 15). The NS5A may be deleted on the C-terminal side (for example, from the 149th amino acid). For example, the NS5A can be NS5A (1-148) having amino acids 1-148 thereof (for example, having a sequence set forth in SEQ ID NO: 16). These factors may be of natural form.

In an aspect, the VAI RNA can be (i) a nucleic acid comprising a sequence set forth in SEQ ID NO: 17, (ii) a nucleic acid consisting of a sequence set forth in SEQ ID NO: 17 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 17, or (iv) a fragment thereof; and it can be a nucleic acid that has the function of inhibiting a PKR (for example, a PKR such as a human PKR or a monkey PKR). According to the examples described below, the reconstitution rates of viruses are improved by inhibiting the PKR in packaging cells. Therefore, the PKR to be inhibited is preferably of the animal species from which the packaging cell is derived. For example, a Vero cell is derived from an African green monkey and an LLC-MK2 cell are derived from a rhesus monkey. Therefore, in these cells, the PKR of the African green monkey and the PKR of the rhesus monkey are preferably inhibited, respectively.

In a preferred aspect, the VAI RNA may have a sequence on the 5'-side of VAI RNA added to the 5'-terminal thereof. In a preferred aspect, the VAI RNA may extend toward the 5'-side to include the 5'-flanking region on the 5'-side. Examples of the sequence to be added to the 5'-terminal include the sequences at positions 1 to 84 of SEQ ID NOs: 19, 20 and 23 to 26, or part thereof that is present contiguously with the VAI RNA. For example, the VAI RNA may have a nucleotide sequence set forth in SEQ ID NO: 34.

In a preferred aspect, the VAI RNA may have a sequence on the 3'-side of VAI RNA added to the 3'-terminal thereof. In a preferred aspect, the VAI RNA may extend toward the 3'-side to include the 3'-flanking region on the 3'-side. Examples of the sequence to be added to the 3'-terminal include the sequences at positions 265 to 330 of SEQ ID NOs: 19, 20 and 23 to 26, or part thereof that is present contiguously with the VAI RNA. For example, the VAI RNA may have a nucleotide sequence set forth in SEQ ID NO: 35.

In a preferred aspect, the VAI RNA may have a sequence on the 5'-side of VAI RNA added to the 5'-terminal thereof and a sequence on the 3'-side of VAI RNA added to the 3'-terminal thereof. In a preferred aspect, the VAI RNA may extend toward the 5'-side and 3'-side to include the 5'-flanking region on the 5'-side and the 3'-flanking region on the 3'-side, respectively. Examples of the sequence to be added to the 5'-terminal include the sequences at positions 1 to 84 of SEQ ID NOs: 19, 20 and 23 to 26, or part thereof that is present contiguously with the VAI RNA. Examples of the sequence to be added to the 3'-terminal include the sequences at positions 265 to 330 of SEQ ID NOs: 19, 20 and 23 to 26, or part thereof that is present contiguously with the VAI RNA.

In a preferred aspect, the VAI RNA is part of SEQ ID NOs: 19, 20 and 23 to 26, and optionally has a sequence comprising either or both of (i) the VAI RNA and (ii) either or both of the sequences on the 5'-side and the 3'-side of VAI RNA, which corresponds to a sequence comprising a nucleotide sequence set forth in SEQ ID NO: 17. For example, the VAI RNA is part of SEQ ID NOs: 19, 20 and 23 to 26 and optionally has a sequence comprising a nucleotide sequence set forth in SEQ ID NO: 17.

In an aspect, the EBER can be (i) a nucleic acid comprising a sequence set forth in SEQ ID NO: 4, (ii) a nucleic acid consisting of a sequence set forth in SEQ ID NO: 4 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 4, or (iv) a fragment thereof; and
it can be a nucleic acid that has the function of inhibiting a PKR (for example, a PKR such as a human PKR or a monkey PKR).

In an aspect, the TAR can be (i) a nucleic acid comprising a sequence set forth in SEQ ID NO: 5, (ii) a nucleic acid consisting of a sequence set forth in SEQ ID NO: 5 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 5, or (iv) a fragment thereof; and
it can be a nucleic acid that has the function of inhibiting a PKR (for example, a PKR such as a human PKR or a monkey PKR).

In an aspect, the 2A^{pro} can be (i) a peptide comprising a sequence set forth in SEQ ID NO: 6, (ii) a peptide consisting of a sequence set forth in SEQ ID NO: 6 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 6, or (iv) a fragment thereof; and
it can be a peptide that has the function of inhibiting a PKR (for example, a PKR such as a human PKR or a monkey PKR).

In an aspect, the E3L can be (i) a peptide comprising a sequence set forth in SEQ ID NO: 7, (ii) a peptide consisting of a sequence set forth in SEQ ID NO: 7 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 7, or (iv) a fragment thereof; and
it can be a peptide that has the function of inhibiting a PKR (for example, a PKR such as a human PKR or a monkey PKR).

In an aspect, the σ3 can be (i) a peptide comprising a sequence set forth in SEQ ID NO: 8, (ii) a peptide consisting of a sequence set forth in SEQ ID NO: 8 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 8, or (iv) a fragment thereof; and
it can be a peptide that has the function of inhibiting a PKR (for example, a PKR such as a human PKR or a monkey PKR).

In an aspect, the p58^{IPK} can be (i) a peptide comprising a sequence set forth in SEQ ID NO: 9, (ii) a peptide consisting of a sequence set forth in SEQ ID NO: 9 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 9, or (iv) a fragment thereof; and
it can be a peptide that has the function of inhibiting a PKR (for example, a PKR such as a human PKR or a monkey PKR).

In an aspect, the K3L can be (i) a peptide comprising a sequence set forth in SEQ ID NO: 10, (ii) a peptide consisting of a sequence set forth in SEQ ID NO: 10 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 10, or (iv) a fragment thereof; and
it can be a peptide that has the function of inhibiting a PKR (for example, a PKR such as a human PKR or a monkey PKR).

In an aspect, the Tat can be (i) a peptide comprising a sequence set forth in SEQ ID NO: 11, (ii) a peptide consisting of a sequence set forth in SEQ ID NO: 11 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 11, or (iv) a fragment thereof; and
it can be a peptide that has the function of inhibiting a PKR (for example, a PKR such as a human PKR or monkey PKR).

In an aspect, the ICP34.5 can be (i) a peptide comprising a sequence set forth in SEQ ID NO: 12, (ii) a peptide consisting of a sequence set forth in SEQ ID NO: 12 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 12, or (iv) a fragment thereof; and
it can be a peptide that has the function of inhibiting a PKR (for example, a PKR such as a human PKR or a monkey PKR).

In an aspect, the nc886 can be (i) a nucleic acid comprising a sequence set forth in SEQ ID NO: 13, (ii) a nucleic acid consisting of a sequence set forth in SEQ ID NO: 13 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 13, or (iv) a fragment thereof; and
it can be a nucleic acid that has the function of inhibiting a PKR (for example, a PKR such as a human PKR or a monkey PKR).

In an aspect, the nc886 can be (i) a nucleic acid comprising a sequence set forth in SEQ ID NO: 14, (ii) a nucleic acid consisting of a sequence set forth in SEQ ID NO: 14 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 14, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 18, (ii) a nucleic acid consisting of a sequence set forth in SEQ ID NO: 18 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 18, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 19, (ii) a nucleic acid consisting of a sequence set forth in SEQ ID NO: 19 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 19, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 20, (ii) a nucleic acid consisting of a sequence set forth in SEQ ID NO: 20 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 20, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 21, (ii) a nucleic acid consisting of a sequence set forth in SEQ ID NO: 21 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 21, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 22, (ii) a nucleic acid consisting of a sequence set forth in SEQ ID NO: 22 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 22, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 23, (ii) a nucleic acid consisting of a sequence set forth in SEQ ID NO: 23 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 23, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 24, (ii) a nucleic acid consisting of a sequence set forth in SEQ ID NO: 24 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 24, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 25, (ii) a nucleic acid consisting of a sequence set forth in SEQ ID NO: 25 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 25, or (iv) a fragment thereof; and

it can be a nucleic acid that has the function of inhibiting a PKR (for example, a PKR such as a human PKR or a monkey PKR).

In an aspect, the VAI RNA can be (i) a nucleic acid comprising a sequence set forth in SEQ ID NO: 26, (ii) a nucleic acid consisting of a sequence set forth in SEQ ID NO: 26 in which 1, 2, 3, 4 or 5 nucleic acids are deleted, substituted, inserted and/or added; or (iii) a nucleic acid comprising a sequence that has 90% or more or 95% or more identity with a sequence set forth in SEQ ID NO: 26, or (iv) a fragment thereof; and

it can be a nucleic acid that has the function of inhibiting a PKR (for example, a PKR such as a human PKR or a monkey PKR).

In an aspect, the NS1 can be (i) a peptide comprising a sequence set forth in SEQ ID NO: 28, (ii) a peptide consisting of a sequence set forth in SEQ ID NO: 28 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 28, or (iv) a fragment thereof; and

it can be a peptide that has the function of inhibiting a PKR (for example, a PKR such as a human PKR or a monkey PKR).

In an aspect, the Us11 can be (i) a peptide comprising a sequence set forth in SEQ ID NO: 29, (ii) a peptide consisting of a sequence set forth in SEQ ID NO: 29 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 29, or (iv) a fragment thereof; and

it can be a peptide that has the function of inhibiting a PKR (for example, a PKR such as a human PKR or a monkey PKR).

In an aspect, E3K3 may be a fusion sequence of part of E3L (for example, the C-terminal 107 amino acids sequence of E3L) or all of E3L and part or all of K3, preferably a fusion sequence of part of E3L (more preferably the C-terminal 107 amino acids sequence of E3L) and K3, and it can be a peptide that has the function of inhibiting a PKR (for example, a PKR such as a human PKR or a monkey PKR). In an aspect, the E3K3 can be (i) a peptide comprising a sequence set forth in SEQ ID NO: 30, (ii) a peptide consisting of a sequence set forth in SEQ ID NO: 30 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 30, or (iv) a fragment thereof; and

it can be a peptide that has the function of inhibiting a PKR (for example, a PKR such as a human PKR or a monkey PKR).

In an aspect, the E3Y3 may be a fusion sequence of part of E3L (for example, the C-terminal 107 amino acids sequence of E3L) or all of E3L and part or all of Y3, preferably a fusion sequence of part of E3L (more preferably the C-terminal 107 amino acids sequence of E3L) and Y3, and it can be a peptide that has the function of inhibiting a PKR (for example, a PKR such as a human PKR or a monkey PKR). In an aspect, the E3Y3 can be (i) a peptide comprising a sequence set forth in SEQ ID NO: 31, (ii) a peptide consisting of a sequence set forth in SEQ ID NO: 31 in which 1, 2, 3, 4 or 5 amino acids are deleted, substituted, inserted and/or added; or (iii) a peptide comprising a sequence that has 90% or more or 95% or more identity with a sequence set forth in SEQ ID NO: 31, or (iv) a fragment thereof; and

it can be a peptide that has the function of inhibiting a PKR (for example, a PKR such as a human PKR or a monkey PKR).

As used herein, the "packaging cell" is a cell that produces a virus vector. Generally, from the viewpoint of improvement in safety, a virus genome of a virus vector is engineered so that factors responsible for proliferation, replication, and spread of the virus vector (including infection to other cells) are disrupted and the virus vector cannot grow, replicate or spread after a cell infection. However, when producing the virus vector, the virus vector is produced while complementing the packaging cell with the disrupted factors in order to enable proliferation, replication, and spread of the virus vector. For this reason, the packaging cells are complemented with the disrupted viral factors in order to cause the packaging cell to produce a virus vector, and the packaging cells are caused to express some of the disrupted viral factors so that the virus production level is restored. The packaging cell may stably possess such factors in the genome or may temporarily possess such factors. In either case, during virus production, the viral factors to be complemented are supplied intracellularly to the packaging cell. As an example, a Sendai virus vector is classically obtained by producing a Sendai virus having a genome deficient in an F gene using a packaging cell that supplies the F gene. The F gene to be supplied is activated in the presence of trypsin, but a type of F gene (F5R) that is activated by furin which is ubiquitous in cells, has also been developed, making virus production more convenient (see, for example, WO 2005/071085A). In recent years, techniques for producing a Sendai virus from cDNA have been developed. For example, vectors, having the strain Z, an attenuated strain as a basic structure, which are designed to further increase safety for medical applications to humans have been constructed. For example, a technique has been developed to delete any one or more of the F, HN, and M genes from a virus genome, thereby causing a loss of viral spread. For example, an F gene-deleted virus genome is preferably used. The virus genome is operably linked to a regulatory sequence (such as a T7 promoter), and the regulatory sequence can drive the production of the virus genome. Thereby, a Sendai virus genome can be produced from cDNA in the packaging cell. When using the T7 promoter as the regulatory sequence (such as a first, second, or third regulatory sequence), a T7 RNA polymerase can be supplied, for example, by a helper virus, such as vaccinia virus. N, P, F, and L operably linked to a regulatory sequence that drives transcription by an RNA polymerase (such as pol II) can be expressed in the packaging cell, thereby supplying a component of a virus particle to form the virus particle in the packaging cell. As the packaging cell, for example, a monkey kidney-derived LLC-MK2 cell is used. This results in a virus particle that can infect a cell once, but cannot spread to other cells thereafter. The virus particle can be used after concentrated and/or purified as necessary. When a foreign gene is integrated into a virus, a regulatory sequence unique to the virus is introduced as necessary to enable transcription by an RNA-dependent RNA polymerase.

According to the present invention, a method of producing a negative-strand RNA virus or a negative-strand RNA virus vector is provided. Examples of the negative-strand RNA virus include the family Orthomyxoviridae (orthomyxoviruses such as influenza virus), the family Paramyxoviridae (paramyxoviruses such as the genus Morbillivirus), the family Rhabdoviridae (rhabdoviruses such as rabies virus), the family Filoviridae (filoviruses such as Ebola virus and Marburg virus) and the family Bunyaviridae (bunyaviruses such as hantavirus). The negative-strand RNA virus may preferably be a virus of the family Paramyxoviridae, preferably a paramyxovirus, and preferably a Sendai virus.

As used herein, the "regulatory sequence" refers to a sequence that has the activity of driving a gene operably linked to it and transcribing RNA from the gene. The regulatory sequence is, for example, a promoter. Examples of the promoter include a class I promoter (which may be used for transcription of an rRNA precursor), class II promoters (composed of a core promoter and upstream promoter elements and which may be used for transcription of an mRNA), and class III promoters (further classified broadly into type I, type II and type III).

In an aspect, the negative-strand RNA virus vector has, on its genome, factors related to proliferation or infection to cells of the negative-strand RNA viruses disrupted (for example, deleted), and has a reduced proliferation or infection ability or no substantial proliferation ability in cells other than the packaging cell. As described above, such a vector is given an initial infectivity to cells by constructing it under conditions in which the packaging cells have been supplied with disrupted growth or infection-related factors. Thereby, the vector obtained from the packaging cell has the infectivity, but the vector that subsequently infects a cell other than the packaging cell cannot generate any more infectious particles, thereby leading to improvement in the safety of the vector.

In an aspect, the method of the present invention comprises:

(A) expressing a protein kinase R (PKR) inhibitory factor from a gene encoding the PKR inhibitory factor operably linked to a first regulatory sequence and supplying it to a packaging cell.

The first regulatory sequence may be a promoter capable of transcribing RNA, such as mRNA, and for example, various pol II-based promoters can be used. Examples of the pol II-based promoter include, but are not limited to, a CMV promoter, an EF1 promoter (an EF1α promoter), an SV40 promoter, an MSCV promoter, an hTERT promoter, a β-actin promoter, a CAG promoter, and a CBh promoter. As the promoter capable of transcribing RNA, a promoter that drives a bacteriophage-derived RNA polymerase, such as a T7 promoter, a T3 promote, or a SP6 promoter and a pol III-based promoter such as U6 promoter can be also used, and the promoter may be preferably a T7 promoter for a circular DNA, and preferably an SP6 promoter for a linear DNA. The promoter may also be an inducible promoter. These promoters can be preferably used for transcription of RNA-based factors. The inducible promoter is a promoter that can induce expression of a polynucleotide functionally linked to the promoter only in the presence of an inducing factor that drives the promoter. Examples of the inducible promoters include a promoter that induces gene expression by heating, such as a heat shock promoter. Examples of the inducible promoter also include a promoter in which the inducing factor that drives the promoter is a drug. Examples of such a drug-inducible promoter include a Cumate operator sequence, a λ operator sequence (such as 12 × λOp), and a tetracycline-inducible promoter. Examples of the tetracycline-inducible promoter include tetracycline or derivatives thereof (such as doxycycline), and a promoter that drives gene expression in the presence of a reverse tetracycline-regulated transactivator (rtTA). Examples of the tetracycline-inducible promoter include a TRE3G promoter.

Certain viruses have a PKR inhibitory viral factor to counteract a PKR. In the present invention, as the PKR inhibitory viral factor, a PKR inhibitory viral factor naturally possessed by such a virus can be used. Therefore, species from which a virus genome and the PKR inhibitory viral factor are derived may be the same, but are preferably different from each other.

Examples of the protein kinase R (PKR) inhibitory factor include a decoy RNA that binds to PKR. Examples of the decoy RNA include VAI RNA, EBER, nc886 and TAR, and orthologs thereof. In a preferred aspect, the decoy RNA may be VAI RNA and orthologs thereof, and particularly one derived from adenovirus. In a preferred aspect, the decoy RNA may be EBER and orthologs thereof and particularly one derived from Epstein-Barr (EB) virus. In a preferred aspect, the decoy RNA may be nc886 and orthologs thereof, and particularly one derived from human. In a preferred aspect, the decoy RNA may be TAR and orthologs thereof, and particularly one derived from human immunodeficiency virus (HIV). These factors can be preferably used in the present invention.

As VAI, one having a sequence set forth in SEQ ID NO: 17 can be used. The VAI may further comprise sequences before and after the above sequence that are on an adenovirus genome. Therefore, the PKR inhibitory viral factor may have a sequence set forth in SEQ ID NO: 19. The VAI may further comprise VAII. For example, the PKR inhibitory viral factor may have a sequence set forth in SEQ ID NO: 21. The VAI may have one or more mutations selected from the group consisting of substitution, deletion, insertion, and additions. For example, the PKR-inhibiting viral factor may have a sequence having a substitution at the base of VAI corresponding to the 74th base in a sequence set forth in SEQ ID NO: 17. The substitution at the 74th base in a sequence set forth in SEQ ID NO: 17 may be substitution with any of G, A, and C, but is preferably substitution with C. In a preferred aspect, the PKR inhibitory viral factor may have a sequence set forth in SEQ ID NO: 18 or 20. In a preferred aspect, the PKR inhibitory viral factor may have a sequence set forth in SEQ ID NO: 22 or 23.

For example, the PKR-inhibiting viral factor may have a sequence having a substitution at the base of VAI corresponding to the 191st base in the sequence set forth in SEQ ID NO: 19. In a preferred aspect, the PKR inhibitory viral factor may have a sequence set forth in SEQ ID NO: 24.

In a preferred aspect, the PKR inhibitory viral factor may have a sequence set forth in SEQ ID NO: 25. In a preferred aspect, the PKR inhibitory viral factor may have a sequence set forth in SEQ ID NO: 26.

In an aspect, the nc886 can have a sequence set forth in SEQ ID NO: 13. The nc886 may further comprise sequences before and after VAI. The nc886 may further have sequences before and after VAI and have a sequence set forth in SEQ ID NO: 14.

Examples of the protein kinase R (PKR) inhibitory factor also include a molecule that induces degradation of PKR. Examples of the molecule that induces degradation of PKR include 2A$^{pro}$ and orthologs thereof. In a preferred aspect, the molecule that induces degradation of PKR may 2A$^{pro}$ and orthologs thereof, and particularly one derived from poliovirus. These factors can be preferably used in the present invention.

Examples of the protein kinase R (PKR) inhibitory factor also include a factor that masks a double-stranded RNA of a virus. The factor that masks a double-stranded RNA of a virus can mask a double-stranded RNA of a virus to prevent activation of PKR. Examples of the factor that masks a double-stranded RNA of a virus include E3L, σ3, Us11, and NS1, and orthologs thereof. In a preferred aspect, the factor that masks a double-stranded RNA of a virus may be E3L and orthologs thereof, and it is particularly one derived from vaccinia virus. In a preferred aspect, the factor that masks a double-stranded RNA of a virus may be σ3 and orthologs thereof, and it is particularly one derived from reovirus. In a preferred aspect, the factor that masks a double-stranded RNA of a virus may be Us11 and orthologs thereof, and it is particularly one derived from herpes simplex virus (HSV). In a preferred aspect, the factor that masks a double-stranded RNA of a virus may be NS1 and orthologs thereof, and it is particularly one derived from influenza virus. These factors can be preferably used in the present invention.

Examples of the protein kinase R (PKR) inhibitory factor also include a factor that inhibits dimerization of PKR. Examples of the factor that inhibits dimerization of PKR include p58$^{IPK}$ and NS5A, and orthologs thereof. In a preferred aspect, the factor that inhibits dimerization of PKR may be p58$^{IPK}$ and orthologs thereof, and it is particularly one derived from human. In a preferred aspect, the factor that inhibits dimerization of PKR may be NS5A and orthologs thereof, and it is particularly one derived from hepatitis C virus (HCV). These factors can be preferably used in the present invention. The NS5A may be deleted on the C-terminal side (for example, from the 149th amino acid onward). For example, the NS5A can be NS5A (1-148) having amino acids 1-148 thereof.

Examples of the protein kinase R (PKR) inhibitory factor also include a pseudosubstrate of PKR. Examples of the pseudosubstrate include K3L and Tat, and orthologs thereof. In a preferred aspect, the pseudosubstrate may be K3L and orthologs thereof, and it is particularly one derived from vaccinia virus. In a preferred aspect, the pseudosubstrate is Tat and orthologs thereof, and particularly one derived from HIV. These factors can be preferably used in the present invention.

Examples of the protein kinase R (PKR) inhibitory factor also include a molecule that induces dephosphorylation of a substrate. Examples of the molecules that induce dephosphorylation of a substrate include ICP34.5 and orthologs thereof. In a preferred aspect, the molecules that induce dephosphorylation of a substrate may be ICP34.5 and orthologs thereof, and it is particularly one derived from herpes simplex virus (HSV). These factors can be preferably used in the present invention.

In an aspect, the protein kinase R (PKR) inhibitory factor is one or more selected from the group consisting of VAI RNA of adenovirus, EBER of EB virus, human nc886, TAR of HIV virus, 2A$^{pro}$ of poliovirus, E3L of vaccinia virus, δ3 of reovirus, NS1 of influenza virus, human p58$^{IPK}$, NS5A of hepatitis C virus, K3L of vaccinia virus, Tat of HIV virus, Us11 of herpes simplex virus and ICP34.5 of herpes simplex virus, and orthologs thereof.

In an aspect, the protein kinase R (PKR) inhibitory factor may be stably integrated into a genome of a packaging cell. The expression can be driven by a first regulatory sequence. The first regulatory sequence may be a constitutive promoter or an inducible promoter.

In an aspect, the protein kinase R (PKR) inhibitory factor may be transiently introduced into a genome of a packaging cell. For example, the protein kinase R (PKR) inhibitory factor can be loaded on a plasmid DNA. The plasmid DNA can be introduced into a cell using any technique well known to those skilled in the art, and the protein kinase R (PKR) inhibitory factor can be thereby expressed in the cell from the plasmid DNA. The expression can be driven by a first regulatory sequence. The first regulatory sequence may be a constitutive promoter or an inducible promoter.

In an aspect, the packaging cell may be a Vero cell or LLC-MK2 cell. In an aspect, the packaging cell may be a cell population consisting of Vero cells or a cell population consisting of LLC-MK2 cells. In an aspect, cells other than the packaging cell are not used for producing a virus. In an aspect, the packaging cell expresses an F gene. In a preferred aspect, the packaging cell constitutively expresses the F gene. In a preferred aspect, F protein can be activated with trypsin. In a preferred aspect, the F protein can be F5R. In a preferred aspect, a composition for reconstitution of SeV comprising a Vero cell or an LLC-MK2 cell is provided. As used herein, the "reconstitution" refers to supplying a component of a virus or virus vector to a packaging cell to form a virus or virus vector. Reconstitution of SeV can be performed as described herein.

According to the present invention, provided is a method of producing a negative-strand RNA virus or a negative-strand RNA virus vector, comprising:
(B) causing the packaging cell to express a genomic RNA of the negative-strand RNA virus or virus vector to form the negative-strand RNA virus or virus vector in the presence of the PKR inhibitory factor {for example, causing the packaging cell to express a genomic RNA of the negative-strand RNA virus or negative-strand RNA virus vector to form the negative-strand RNA virus or negative-strand RNA virus vector in the presence of the PKR inhibitory factor such as a PKR inhibitory viral factor} .

The genomic RNA of the negative-strand RNA virus or negative-strand RNA virus vector can be expressed, for example, from a gene expression vector having DNA encoding the genomic RNA operably linked to a second regulatory sequence. As the gene expression vector to be used, for example, any gene expression vector that at least transiently expresses the genomic RNA in a cell can be used, but for example, in a preferred example, it may be a plasmid vector. The genomic RNA comprises the mutation of 511F in the P protein, the mutations of 69E, 116A and 183S in the M protein, the mutations of 262T, 264R and 461E in the HN protein, and the mutations of 1197S and 1796E in the L protein to minimize the cytotoxicity of the vector (see, for example, International Publication No. WO 2003/025570). When the genomic RNA is produced in the cell, it is complexed with other virus particle components (for example, F, N, P, and L) supplied to the packaging cell to form a virus or virus vector (virus particle). At this time, if the protein kinase R (PKR) inhibitory factor, NS5A or nc886, or equivalents thereof are present in the cell, the production level of virus particles increases. The packaging cell can be cultured under conditions suitable for culture. The gene encoding the virus genome can be driven by a T7 promoter. In this case, the packaging cell can be supplied with a T7 polymerase. The gene encoding the virus genome may be driven by a CAG promoter or an EF1 promoter.

In an aspect, the packaging cell can be further supplied with components of SeV (for example, N, P and L, and equivalents thereof) to promote formation of the SeV in the packaging cell. The components of SeV can be supplied, for example, by introducing a plasmid vector into the packaging cell.

In an aspect, a gene encoding components of a virus particle (for example, N, P and L) are each operably linked to a third regulatory sequence. In an aspect, the gene encoding components of a virus particle (for example, F, N, P and L), each operably linked to a third regulatory sequence, is integrated on a plasmid. In an aspect, the third regulatory sequence may be a CAG promoter (for example, having a sequence set forth in SEQ ID NO: 2). In an aspect, at least one or all of the third regulatory sequences can be a non-CAG promoter, such as an EF1α promoter (for example, having a sequence set forth in SEQ ID NO: 1). In a preferred aspect, the gene encoding components of a virus particle (for example, N, P and L) are loaded on one or more gene expression vectors (preferably plasmids). In this case, these components may be operably linked to one regulatory sequence or to a plurality of regulatory sequences. The regulatory sequence may be, for example, a CAG or EF1 promoter.

In an aspect of the present invention, the method of producing a negative-strand RNA virus or a negative-strand RNA virus vector further comprises:

(C) recovering the formed negative-strand RNA virus or negative-strand RNA virus vector.

The virus particle formed in the cell can be recovered as appropriate. The virus particle formed in the cell may be released outside the cell. Therefore, the virus particle can be recovered from a culture medium. The recovered virus particle can be purified and/or concentrated as appropriate. In this way, the isolated virus vector, purified virus vector, or concentrated virus vector is provided. The resulting virus vector may be stored as appropriate. The storage can be performed, for example, in a deep freezer (for example, at a temperature of about -80°C), in a freezer (for example, at a temperature of about -20°C), or in a refrigerator (at a temperature of about 4°C). The storage can also be performed in liquid nitrogen. The virus vector is subjected to titer measurement as necessary. The virus titer can be determined by any method well known to those skilled in the art.

In an aspect, the infectious titer of the negative-strand RNA virus or negative-strand RNA virus vector obtained by the method of the present invention may be $1 \times 10^5$ CIU/mL or more, $2 \times 10^5$ CIU/mL or more, $3 \times 10^5$ CIU/mL or more, $4 \times 10^5$ CIU/mL or more, $5 \times 10^5$ CIU/mL or more, $6 \times 10^5$ CIU/mL or more, $7 \times 10^5$ CIU/mL or more, $8 \times 10^5$ CIU/mL or more, $9 \times 10^5$ CIU/mL or more, $1 \times 10^6$ CIU/mL or more, $2 \times 10^6$ CIU/mL or more, $3 \times 10^6$ CIU/mL or more, $4 \times 10^6$ CIU/mL or more, $5 \times 10^6$ CIU/mL or more, $6 \times 10^6$ CIU/mL or more, $7 \times 10^6$ CIU/mL or more, $8 \times 10^6$ CIU/mL or more, $9 \times 10^6$ CIU/mL or more, $1 \times 10^7$ CIU/mL or more, $2 \times 10^7$ CIU/mL or more, $3 \times 10^7$ CIU/mL or more, $4 \times 10^7$ CIU/mL or more, or $5 \times 10^7$ CIU/mL or more.

In an aspect of the present invention, the relationship between the species from which the virus or virus vector is derived and the species from which the PKR inhibitory factor is derived may be heterologous. In this aspect, the relationship between the species from which the first regulatory sequence is derived and the species from which the PKR inhibitory factor is derived may be homologous.

In an aspect of the present invention, the relationship between the species from which the first regulatory sequence is derived and the species from which the PKR inhibitory factor is derived may be heterologous. In this aspect, the relationship between the species from which the virus or virus vector is derived and the species from which the PKR inhibitory factor is derived may be homologous.

In an aspect of the present invention, the relationship between the species from which the virus or virus vector is derived and the species from which the PKR inhibitory factor is derived may be heterologous, and the relationship between the species from which the first regulatory sequence is derived and the species from which the PKR inhibitory factor is derived may be heterologous.

In an aspect of the present invention, a gene encoding the PKR inhibitory factor is integrated into the genome of a packaging cell. In an aspect of the present invention, the gene encoding the PKR inhibitory factor is integrated into a vector (such as a plasmid DNA) to be introduced into a packaging cell. The site of integration is before an N gene, between the N gene and a P gene, between the P gene and an M gene, between the M gene and an HN gene, between the HN gene and an L gene, or after the L gene, on an SeV genome. When the factor is an RNA, the site of integration may be in the 3'UTR of a gene encoding a protein of interest. In general, the N gene is sometimes referred to as the NP gene.

In a preferred aspect of the present invention, the gene encoding the PKR inhibitory factor is integrated into the RNA genome of the negative-strand RNA virus or the negative-strand RNA virus vector. In this aspect, the gene encoding the PKR inhibitory factor may be further integrated into a genome of the packaging cell or a vector (such as a plasmid DNA) to be introduced into the packaging cell. Therefore, in a preferred aspect, the gene encoding the PKR inhibitory factor is integrated into an RNA genome of the negative-strand RNA virus or the negative-strand RNA virus vector, and it is further integrated into a genome of the packaging cell or into a vector (such as a plasmid DNA) to be introduced into the packaging cell. Thereby, even when the number of genomes is small, the PKR inhibitory factor is supplied from the packaging cell, and when the number of genomes increases, the PKR inhibitory factor is supplied also from the genome, so that the negative-strand RNA virus or the negative-strand RNA virus vector may increase satisfactorily. In addition, even after infecting another cell (non-packaging cell) with the obtained negative-strand RNA virus or virus vector, the PKR inhibitory factor is supplied from the virus genome, so that the virus or virus vector can grow in the cell satisfactorily. Thereby, for example, the effect of increasing the expression level of the gene of interest loaded on the virus or virus vector can be obtained.

In an aspect of the present invention, the virus particle is produced in the absence of a helper virus.

In an aspect of the present invention, the virus or virus vector is a paramyxovirus or a paramyxovirus vector, and the RNA genome is an F gene-deficient RNA genome. In this aspect, the first regulatory sequence may be a CAG promoter or an EF1 promoter, for example, an EF1 promoter. In this aspect, in addition, the second regulatory sequence is a T7 promoter, and a T7 RNA polymerase can be produced by transcription from the cell genome or plasmid and translation. In a preferred aspect, the paramyxovirus is a Sendai virus. In a preferred aspect, the paramyxovirus vector is a Sendai virus vector.

In an aspect, the paramyxovirus or paramyxovirus vector can express N, P, and L proteins. In an aspect, the paramyxovirus or paramyxovirus vector does not express one, two, or three proteins selected from the group consisting of F, HN, and M proteins. For example, the RNA genome is deleted in the RNA encoding one, two, or three proteins selected from the group consisting of F, HN, and M proteins. In a preferred aspect, the paramyxovirus or paramyxovirus vector does not express the F protein.

In an aspect, the paramyxovirus or paramyxovirus vector has an RNA genome comprising expressibly V protein and/or C protein.

According to the present invention, an RNA genome of a negative-strand RNA virus or a negative-strand RNA virus vector and a DNA encoding the RNA genome are provided. In an aspect, the RNA genome comprises expressibly a gene encoding any one or more of PKR-inhibiting factors (such as PKR-inhibiting viral factors). According to the present invention, a negative-strand RNA virus vector comprising the RNA genome is provided. The negative-strand RNA virus or negative-strand RNA virus vector further has a virus particle that contains an RNA genome. Such a virus or virus vector may exhibit stronger proliferative ability after infecting cells. In a preferred aspect, the negative-strand RNA virus is a paramyxovirus, and more preferably a Sendai virus. In a preferred aspect, the negative-strand RNA virus vector may be a paramyxovirus vector, and more preferably a Sendai virus vector. In an aspect, the DNA encoding the RNA genome is operably linked to a second regulatory sequence. In an aspect, provided is a gene expression vector comprising a DNA encoding the RNA genome operably linked to the second regulatory sequence.

In a preferred aspect, the gene expression vector of an RNA genome (particularly an SeV genome), comprising DNA encoding the genome may comprise a self-cleaving ribozyme (such as a hammerhead ribozyme or HDV ribozyme), flanking either or both upstream and/or downstream of the RNA genome. In addition, the gene expression vector may have a sequence of the other factor (such as a PKR inhibitory factor or a factor that promotes RNA amplification) linked through the sequence of the self-cleaving ribozyme (such as a hammerhead ribozyme or HDV ribozyme), whereby the sequences of the RNA genome and the other factor may be transcribed into a strand of RNA. With such a constitution, after transcription of the RNA genome, the RNA can be cleaved at the sequence of the self-cleaving ribozyme (such as a hammerhead ribozyme or HDV ribozyme) to remove the other factor, so that the RNA consisting substantially of the RNA genome can be obtained. By linking, for example, the PKR inhibitory factor or the factor that promotes RNA amplification as a sequence of the other factor, after exerting its effect and amplifying the genome, the other factor can be removed from the completed genome. For example, the other factor can be positioned on the 5' side of the RNA genome and a sequence of a hammerhead ribozyme can be positioned between the other factor and the region encoding the RNA genome, or alternatively, the other factor can be positioned on the 3' side of the RNA genome and a sequence of a HDV ribozyme can be positioned between the region encoding the RNA genome and the other factor. More specifically, the gene expression vector comprising a DNA encoding the RNA genome may be, for example, configured to comprise, in the following order, a promoter (such as a T7 promoter, a CAG promoter or an EF1 promoter), a sequence of a first self-cleaving ribozyme (such as a 3' self-cleaving ribozyme, preferably a hammerhead ribozyme), a DNA encoding the RNA genome, a sequence of a second self-cleaving ribozyme (such as a 5' self-cleaving ribozyme, preferably an HDV ribozyme) and a sequence of the other factor. In another preferred aspect, the gene expression vector comprising a DNA encoding the RNA genome may be configured to comprise, in the following order, a promoter (such as a T7 promoter, a CAG promoter or an EF1 promoter), a sequence of the other factor, a sequence of a first self-cleaving ribozyme (such as a 3' self-cleaving ribozyme, preferably a hammerhead ribozyme), a DNA encoding the RBA genome and a sequence of a second self-cleaving ribozyme (such as a 5' self-cleaving ribozyme, preferably an HDV ribozyme). In the above, the self-cleaving ribozyme is described only as an example, and preferred embodiments are not limited thereto. In particular, when using a self-cleaving ribozyme, the self-cleaving ribozyme can be arranged so that the number of bases in the RNA genome is 6n {wherein n is a natural number}. The gene expression vector may further comprise, for example, a terminator sequence (for example, a T7 terminator sequence for a T7 polymerase) downstream of the sequence of the other factor. Various sequences are known as the sequence of the self-cleaving ribozyme (such as hammerhead ribozyme). Examples of the self-cleaving ribozyme include, but are not limited to, a hammerhead ribozyme, a hepatitis delta virus (HDV) ribozyme, a twister ribozyme, a twister-sister ribozyme, a pistol ribozyme, a hairpin ribozyme, and a hatchet ribozyme. These self-cleaving ribozymes may be, for example, 5' self-cleaving ribozymes and/or 3' self-cleaving ribozymes. Among them, the sequence of Hh-Rbz (for example, SEQ ID NO: 36) can be used as the 3' self-cleaving ribozyme, and the sequence of a hepatitis delta virus ribozyme (HDV-Rbz) (for example, SEQ ID NO: 37) and the like can be used as the 5' self-cleaving ribozyme.

In a preferred aspect, the gene expression vector expressing a constituent factor of an RNA virus may further have, a PKR inhibitory factor or a factor that promotes RNA amplification, loaded hereon. The gene expression vector may have a sequence of the other factor (such as a PKR inhibitory factor or a factor that promotes RNA amplification) linked to the constituent factor through a sequence of the self-cleaving ribozyme (for example, it is as described above and can be, for example, a hammerhead ribozyme or an HDV ribozyme), whereby the sequences of the RNA genome and the other factor may be transcribed into a strand of RNA. Alternatively, in a preferred aspect, the PKR inhibitory factor or the factor that promotes RNA amplification may be independently loaded on a plasmid.

According to the present invention, provided is a combination of a gene expression vector of the RNA genome of the virus comprising a DNA encoding the RNA genome of the virus and a vector for reconstitution of the virus comprising a DNA encoding a constituent factor of the virus. The combination can be used to reconstitute the virus particle in a cell by coexpression in the cell. In an aspect, provided is a combination for use in reconstituting a virus particle; wherein the combination comprises a DNA encoding the RNA genome of the virus and a DNA encoding a constituent factor of the virus; and these DNAs comprise DNAs encoding the factors that are sufficient to constitute the virus particle, and are loaded on one or more gene expression vectors. The DNAs encoding the RNA genome and the constituent factor of the virus are operably linked to a regulatory sequence. The regulatory sequence may be a promoter, examples of which include, but are not limited to, a T7 promoter, a CAG promoter and an EF1 promoter.

The Sendai virus or Sendai virus vector may further have a gene of interest in the RNA genome thereof. The gene of interest may be a gene to be introduced into a cell. The gene of interest can be expressed in the cell into which it has been introduced to produce an RNA or a protein. The gene of interest is expressibly loaded on the RNA genome. The gene of interest may be a foreign gene. The foreign gene is a term used to distinguish it from a gene that is endogenous to a cell into which it is introduced.

Examples

Preparation of negative-strand RNA virus vector

A negative-strand RNA virus vector was expressed in a viral packaging cell. In particular, the effectiveness of PKR inhibitory factors at the time of expression was evaluated.

A Sendai virus (SeV) was used as the negative-strand RNA virus vector. The Sendai virus was recovered from a culture supernatant three days after introducing a plasmid mixture into an F gene-expressing cell. The plasmid mixture contained pSeV-EmGFP on which a Sendai virus genome operably linked to a T7 promoter was loaded. The Sendai virus genome was loaded with a gene encoding EmGFP so that the proliferation of the genome was able to be detected by fluorescence. The Sendai virus genome was one deleted in the F gene.

(1) The F gene-expressing cell was constructed as follows.
An F gene (having Kozak sequence added and being optimized to a human codon) of the SeV was loaded on pCAGGS-neo to construct pCAGGS-F-neo. A Vero cell or LLC-MK2 cell, using ViaFect (Promega), was transfected with the obtained plasmid and selected using 1 to 2 mg/mL G418 disulfate solution (NACALAI TESQUE, INC.) to obtain an F gene-expressing cell. Each of the obtained cells is represented as Vero-F or LLC-MK2-F.

(2) An F gene-deleted SeV was constructed as follows.

pSeV/dF in which the F gene-deleted SeV genome is to be transcribed with a T7 promoter was constructed on the basis of the gene sequence information of the SeV-Z strain of ACCESSION: AB855655 and the information described in J. General Virology (1997), 78, 2813 - 2820. The following amino acid mutations were added to pSeV/dF in order to minimize the cytotoxicity of the SeV-Z strain: P protein: 511F, M protein: 69E, 116A and 183S; HN protein: 262T, 264R and 461E; and L protein: 1197S and 1796E (see, for example, WO 2003/025570). The obtained plasmid was referred to as pSeV/TSdF. EmGFP, a mutant of GFP, was used as a gene of interest (GOI) to evaluate SeV reconstitution. The gene expression level decreases depending on the GOI-loaded position in the following order: before an N gene (hereinafter designated as "+"), between a P gene and an M gene (hereinafter designated as "PM"), and between the M gene and an HN gene (hereinafter designated as "MHN") and between the HN gene and an L gene (hereinafter designated as "HNL"). The EmGFP gene was loaded before the N gene of SeV, and pSeV+EmGFP/TSdF was mainly used.

(3) Plasmids for SeV reconstruction were prepared as follows.

pCAGGS-NP, pCAGGS-P4C(-), pCAGGS-L, pCAGGS-F5R and pCAGGS-T7 were constructed with reference to WO 2005/071092. This combination is designated as "CAG". In addition, the addition of a Kozak sequence and optimization to a human codon were performed, and the following reconstitution plasmid set with a promoter different from that described above was constructed: pCAGGS-NPco, pCAGGS-P4C(-)co, pCAGGS-Lco, pCAGGS-F5Rco, pCAGGS-T7co, pEF1-NPco, pEF1-P4C(-)co, pEF1-Lco, pEF1-F5Rco and pEF1-T7co. Furthermore, 430P, 849I, and 880Y mutations were introduced into T7 with reference to P2001-54387A, and 644Y and 667Y mutations were introduced into T7 with reference to P2003-61683A, and pCAGGS-T7mco and pEF1-T7mco having the resulting T7m sequence loaded thereon were constructed. The combination of pEF1-NPco, pEF1-P4C(-)co, pEF1-Lco, pCAGGS-F5Rco, and pCAGGS-T7mco is designated as "EFnpL". The combination of pEF1-NPco, pCAGGS-P4C(-)co, pCAGGS-Lco, pCAGGS-F5Rco, and pCAGGS-T7mco is designated as "EFnCAGpL". The combination of pCAGGS-NPco, pCAGGS-P4C(-)co, pEF1-Lco, pCAGGS-F5Rco, and pCAGGS-T7mco is designated as "CAGnpEFL".

(4) Plasmids having a PKR inhibitory factor were constructed as follows.

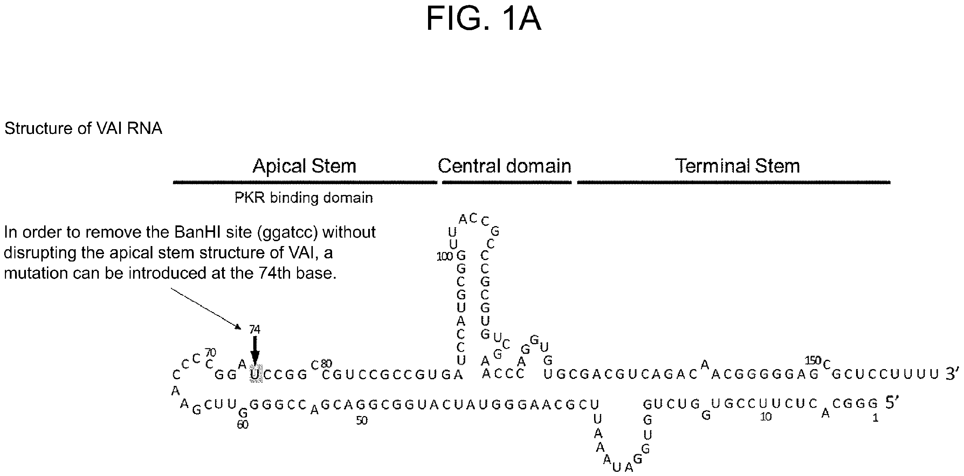

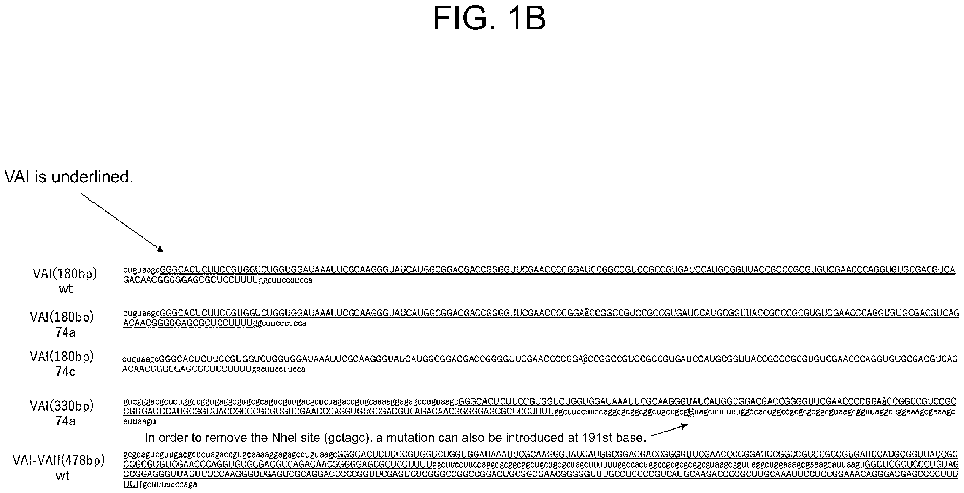

Sequences for evaluating non-coding RNAs had a pT7 plasmid having a T7 promoter and a T7 terminator loaded thereon, and the following were constructed: pT7-VAI (180 bp; SEQ ID NO: 17), pT7-VAI74a (180 bp; V = A in SEQ ID NO: 18), pT7-VAI74c (180 bp; V = C in SEQ ID NO: 18), pT7-VAI74a (330bp; M = A in SEQ ID NO: 26), pT7-VAI-VAII (478bp; SEQ ID NO: 21), pT7-nc886 (108 bp; SEQ ID NO: 13) and pT7-nc886 (272 bp; SEQ ID NO: 14) were constructed. In order to disrupt the BamHI recognition sequence in the Apical Stem of VAI (Figure 1), sequences with a base substitution (T74A and T74C) at the 74th base of VAI were constructed. Similarly, in order to disrupt the NheI recognition sequence on the 3' side of VAI, a sequence with a base substitution (G191C) at the 191st base was constructed (M = A in SEQ ID NO: 26). These base substitutions were designed on the assumption that they would not affect the secondary structure of RNA and would not affect the function of VAI. A pT7 plasmid having nc886 (108 bp) loaded thereon as another PKR inhibitory factor was constructed. In order to extend the sequence, nc886 (272 bp) was constructed which further, before and after it, has sequences before and after VAI, respectively (SEQ ID NOs: 13, 14). pT7-IRES having an IRES sequence under T7 promoter was constructed as a control plasmid.

(5) Plasmids containing a PKR inhibitory factor were constructed as follows.

Basically, a sequence to be translated was subjected to addition of a Kozak sequence and optimization to a human codon. pCAGGS-E3L, pCAGGS-K3L, pCAGGS-Y3, pCAGGS-E3K3, pCAGGS-E3Y3, pCAGGS-NS1, pCAGGS-σ3 and pCAGGS-Us11 were constructed. E3L (SEQ ID NO: 7) and K3L (SEQ ID NO: 10) were sequences of vaccinia virus; Y3 (SEQ ID NO: 27) was the C-terminal 106 amino acid sequence of SeV C protein; NS1 (SEQ ID NO: 28) was the sequence of influenza virus; σ3 (SEQ ID NO: 8) was the sequence of reovirus; Us11 (SEQ ID NO: 29) was the sequence of HSV-1; E3K3 (SEQ ID NO: 30) was a fusion sequence of the C-terminal 107 amino acid sequence of E3L and K3L; and E3Y3 (SEQ ID NO: 31) was a fusion sequence of the C-terminal 107 amino acid sequence of E3L and Y3.

(6) Reconstitution of SeV in the presence or absence of PKR inhibitor

The ratio of the plasmid for SeV reconstruction and a transfection reagent was in accordance with WO 2005/071092. Specifically, the following weights of the plasmid and the transfection reagent (TransIT-LT1 Reagent or ViaFect) were mixed to obtain a plasmid mix.

Conditions in which no PKR inhibitory factor is contained:

NP, P4C(-), F5R, T7: 0.5 μg each

L: 2 μg
pSeV: 5 μg
Total plasmids: 9 μg
TransIT-LT1 Reagent or ViaFect: 15 μL

Conditions in which a PKR inhibitory factor is contained:

NP, P4C(-), F5R, T7: 0.5 μg each
L: 2 μg
pSeV: 5 μg
Plasmid containing PKR inhibitory factor: 1 μg
Total plasmids: 10 μg
TransIT-LT1 Reagent or ViaFect: 16.5 μL

Hereinafter, unless otherwise specified, the plasmid and the volume of the transfection reagent were mixed to provide a ratio of the weight of the plasmid and the volume of the transfection reagent of 9:15.

The plasmid and the transfection reagent were mixed into 225 μL of Opti-MEM, and the mixture was transfected into an F gene-expressing cell in a 12-well plate, which was being cultured in 500 μL of a medium (10% FBS/E-MEM), and was cultured at 37°C. The cells were cultured at 32°C from the day after transfection. The culture medium was exchanged daily with a serum-free medium (ITS-X/NEAA/E-MEM) supplemented with 2.5 μg/mL trypsin. As an indicator of successful SeV reconstitution, EmGFP-positive cells were observed from the day after transfection (an increase in the number of the EmGFP-positive cells indicates an improvement in the SeV reconstitution efficiency), and the fluorescence intensity of the microplate was measured using a fluorescence microscope system ECLIPSE Ti2-E (NIKON CORPORATION) on the third day of transfection. The culture supernatant on the third day after transfection was recovered, diluted 10 times to 100,000 times, and a Vero cell that had been seeded in a 96-well plate was infected therewith. The number of GFP-positive cells was counted 3 days after infection using ECLIPSE Ti2-E, and the infectious titer was calculated.

(7) Reconstitution of SeV in the presence of VAI

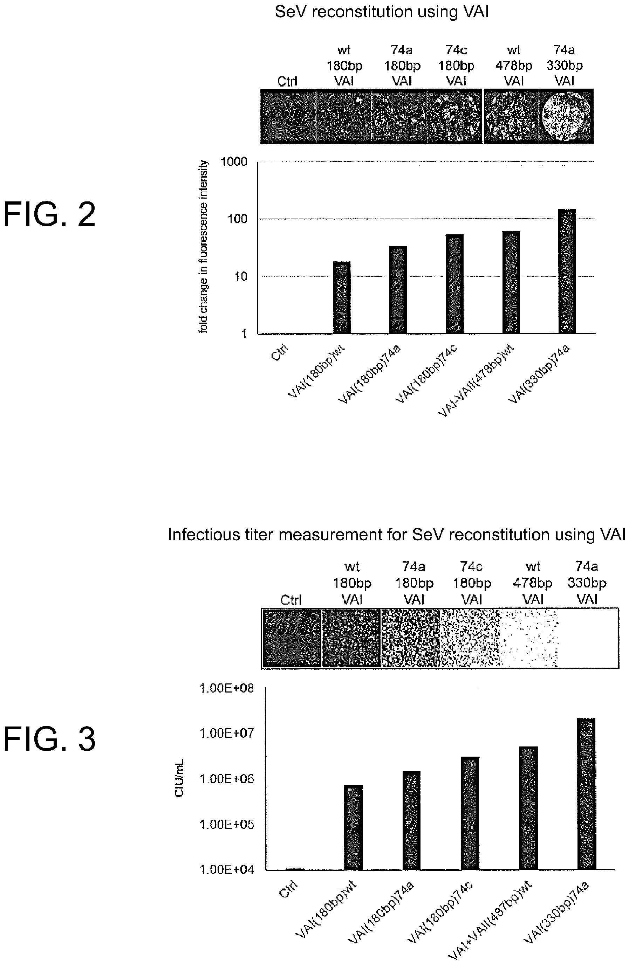

A cell population containing only LLC-MK2-F was used as cells for SeV reconstitution. CAGnpEFL was used as a plasmid for SeV reconstruction. pSeV+EmGFP/TSdF was used as pSeV. VAI was used as a PKR inhibitory factor. For VAI, pT7-VAI (180 bp) and pT7-VAI-VAII (478 bp) were used as wild type (wt) sequences, and pT7-VAI74a (180 bp), pT7-VAI74c (180 bp) and pT7-VAI74a (330 bp) were used as base substitution sequences. pT7-IRES was used as a control (Ctrl) plasmid for VAI. In cells used in combination with VAI, the EmGFP fluorescence-positive cells were observed from the day after transfection unlike the case of using the control. Comparing the fluorescence intensities derived from Em-GFP from cells on the third day, the fluorescence intensity was 18 times that of the control in the presence of VAI (180 bp) wt, 34 times that of the control in the presence of VAI (180 bp) 74a, and 53 times that of the control in the presence of VAI (180 bp) 74c, 63 times that of the control in the presence of VAI-VAII, and 146 times that of the control in the presence of VAI (330 bp) 74a, indicating that when used in combination with VAI, the number of EmGFP-positive cells significantly increased (Figure 2). The infectious titer of the culture supernatant on the third day after the start of SeV reconstitution to the Vero cell was 68 times that of the control in the presence of VAI (180 bp) wt, 139 times that of the control in the presence of VAI (180 bp) 74a, 282 times that of the control in the presence of VAI (180 bp) 74c, 476 times that of the control in the presence of VAI-VAII, and 1,962 times that of the control in the presence of VAI (330 bp) 74a (Figure 3). It is thus clear that VAI significantly increases the SeV reconstitution efficiency.

According to WO 2005/071092, it is shown that the recovery efficiency of SeV vectors was very low in LLC-MK2 and that even if the recovered cells were inoculated into a chicken egg, the HA activity of SeV was not able to be confirmed when the vectors were obtained in cells derived from LLC-MK2. The reconstitution of SeV with a 293T cell showed an infectious titer of about $10^2$ CIU/mL when the culture supernatant on the third day after reconstitution was used (Beaty, SM. et al., mSphere. 2, e00376 - 16 (2017)). In contrast, in the present Example, the infectious titer of $2 \times 10^7$ CIU/mL was achieved on the third day after reconstitution, indicating that the infectious titer significantly increased.

(8) Reconstitution of SeV in the presence of nc886

A cell population containing only LLC-MK2-F was used as cells for SeV reconstitution. CAGnpEFL was used as a plasmid for SeV reconstitution. pSeV+Em-GFP/TSdF was used as pSeV. pT7-nc886 (108 bp), pT7-nc886 (272 bp) and pT7-VAI74a (330 bp) were used as plasmids containing a PKR inhibitory factor.

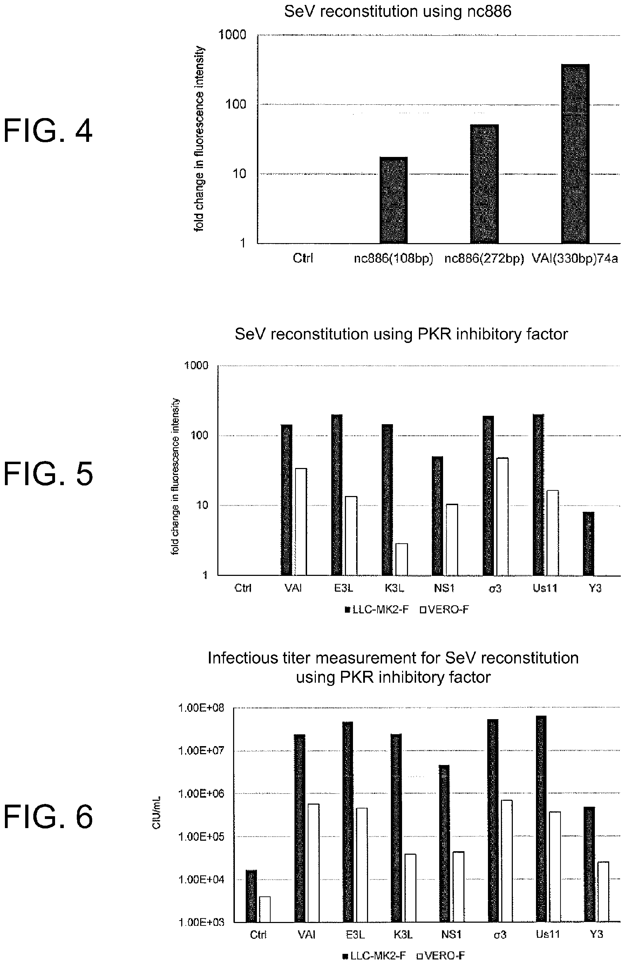

Comparing the fluorescence intensities derived from EmGFP from cells on the third day after reconstitution, the fluorescence intensity was 17 times that of the control in the presence of nc886 (108 bp), 52 times that of the control in the presence of nc886 (272 bp), and 381 times that of the control in the presence of VAI (330 bp) 74a (Figure 4).

(9) Reconstitution of SeV in the presence of PKR-inhibitory viral factor

A cell population containing only LLC-MK2-F or cells containing only Vero-F was used as cells for SeV reconstitution. CAGnpEFL was used as a plasmid for SeV reconstitution. pSeV+EmGFP/TSdF was used as pSeV. Any of pT7-VAI74a (330 bp), pCAGGS-E3L, pCAGGS-K3L, pCAGGS-NS1, pCAGGS-σ3, pCAGGS-Us11 and pCAGGS-Y3 was used as a PKR inhibitory plasmid. The fluorescence intensity derived from Em-GFP from the cells on the third after reconstitution was measured. As shown in Figure 5, the fluorescence intensity derived from EmGFP from the cells increased in all test groups compared to that of the control. VAI, E3L, σ3 and Us11 improved the SeV reconstitution efficiency by 100 times or more in the LLC-MK2-F cells and by 10 times or more in the VERO-F cells compared to that of the control (Figure 5). In addition, as shown in Figure 6, the infectious titer increased in all test groups compared to that of the control. In titer measurement, VAI, E3L, σ3 and Us11 showed high values in both the LLC-MK2-F and Vero-F cells (Figure 6).

(10) Reconstitution of SeV in the presence of fusion sequence

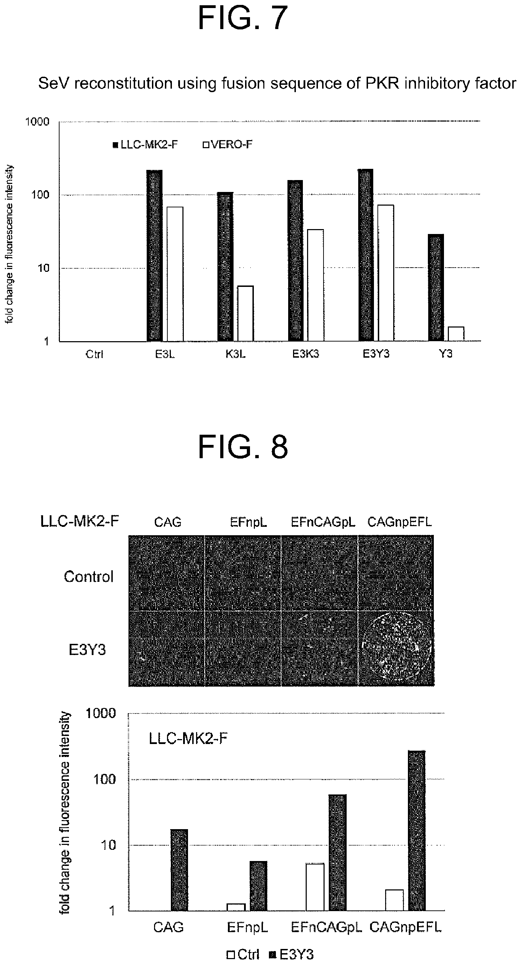

A cell population containing only LLC-MK2-F or cells containing only Vero-F were used as cells for SeV reconstitution. CAGnpEFL was used as a plasmid for SeV reconstruction. pSeV+EmGFP/TSdF was used as pSeV. Any of pCAGGS-E3L, pCAGGS-K3L, pCAGGS-E3K3, pCAGGS-E3Y3 and pCAGGS-Y3 was used as a PKR inhibitory plasmid. The fluorescence intensity derived from EmGFP from the cells on the third after reconstitution was measured. Then, as shown in Figure 7, the fluorescence intensity derived from EmGFP from the cells increased in all test groups compared to that of the control. E3Y3 improved SeV reconstitution efficiency compared to E3K3 (Figure 7). When EFnpL was used as a plasmid for SeV reconstitution and the SeV reconstitution efficiencies were compared on the third day after infection, the SeV reconstitution efficiency in the LLC-MK2-F cells was observed to be improved for E3Y3 by 3.5 times over E3L, and the SeV reconstitution efficiency in the Vero-F cells was observed to be improved for E3Y3 by 6.8 times over E3L.

(11) Reconstitution of SeV using cells that constitutively express PKR inhibitory factor

Vero-F cells were transfected with pCAGGS-E3L-Hyg or pCAGGS-E3Y3-Hyg, the cells were selected using 500 μg/mL hygromycin B (NACALAI TESQUE, INC.) to obtain Vero-F-E3L and Vero-F-E3Y3 cells, which are of stable cell lines that constitutively express E3L and E3Y3, respectively. The combination of plasmids for SeV reconstitution, pEF1-NPco, pEF1-P4C(-)co, pEF1-Lco, pCAGGS-F5Rco and pCAGGS-T7mco (EFnpL) and pSeV+EmGFP/TSdF were used for these cells. The fluorescence intensity derived from EmGFP from the cells on the third after reconstitution was measured. The SeV reconstitution efficiency in the Vero-F-E3Y3 cells was observed to be improved by twice.

(12) Comparison of expression promoters

The combination of a CAG promoter and an EF1 promoter was investigated for comparison to the conventional technology. LLC-MK2-F and Vero-F were used as cells. pSeV+EmGFP/TSdF was used as pSeV. E3Y3 was used as a PKR inhibitory factor. With the combination of CAG that drives all N, P and L from the CAG promoter; EFnpL that drives all N, P and L from the EF1α promoter; EFnCAGpL that drives N from the EF1α promoter and P and L from the CAG promoter; and CAGnpEFL that drives N and P from the CAG promoter and drives L from the EF1α promoter, the effect of different promoters on the SeV reconstitution efficiency was examined. The fluorescence intensity derived from EmGFP from the cells on the third day after reconstitution was measured.

As a result, in LLC-MK2 cells, as shown in Figure 8, codon optimization of the plasmid used for SeV reconstitution alone did not result in a sufficient improvement in the SeV reconstitution efficiency (see CAG in the absence of a PKR inhibitory factor E3Y3). In contrast, SeV reconstitution was observed with sufficient efficiency in the presence of E3Y3, even when driven by any promoter. In the system in which N and P were expressed with the CAG promoter and L was expressed with the EF1a promoter, in the presence of the PKR inhibitory factor E3Y3, the SeV reconstitution efficiency was improved to 271 times compared to CAG (a conventional plasmid).

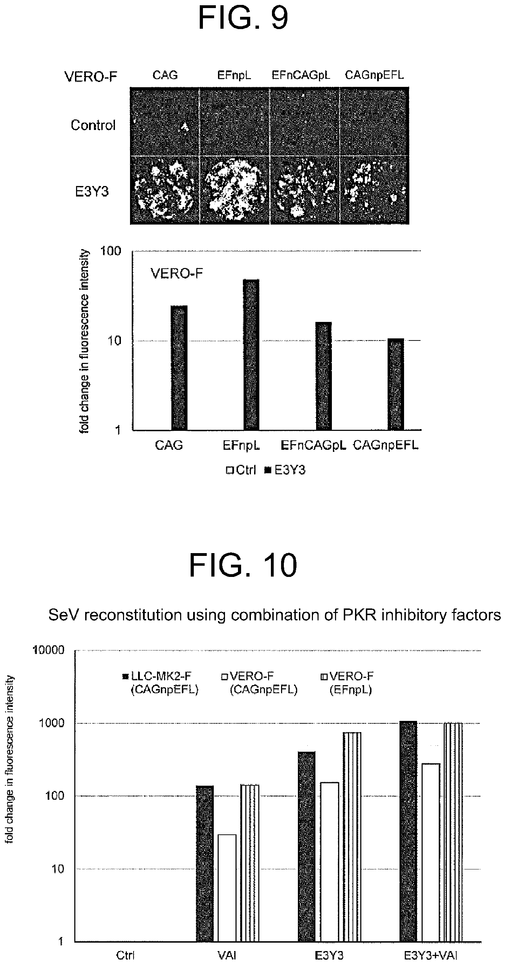

In Vero cells, as shown in Figure 9, in the presence of the PKR inhibitory factor E3Y3, the reconstitution efficiency was improved with any promoter, but the SeV reconstitution efficiency was improved to 48 times compared to CAG (a conventional plasmid), by expressing N, P and L with the EF1 promoter.

(13) Reconstitution of SeV in the presence of the combination of PKR inhibitory factors

A cell population containing only LLC-MK2-F or a cell population containing only Vero-F were used as cells for SeV reconstitution. pSeV+EmGFP/TSdF was used as pSeV. VAI and E3Y3 were used as PKR inhibitory factors. CAGnpEFL was used for LLC-MK2-F, and CAGnpEFL and EFnpL were used for Vero-F, as plasmids for SeV reconstitution. As a result, as shown in Figure 10, the combination of the PKR inhibitory factors improved the SeV reconstitution efficiency to 1000 times or more compared to that of the control in the absence of the PKR inhibitory factor.

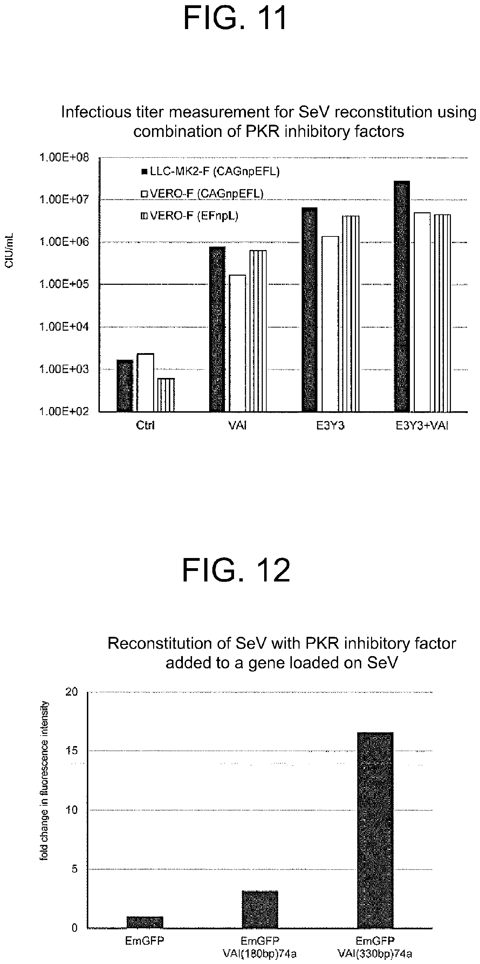

Furthermore, as shown in Figure 11, the infectious titer of the culture supernatant on the third day after the start of SeV reconstitution was improved to 16,857 times in the reconstitution system of the LLC-MK2-F cells and to 7,460 times in the reconstitution system of the VERO-F cells, compared to that of the control in the absence of the PKR inhibitory factor.

(14) Reconstitution of SeV loaded with PKR inhibitory factor

The SeV reconstitution efficiency was evaluated for SeV loaded with a gene encoding a PKR inhibitory factor. A cell population containing only LLC-MK2-F was used as cells. CAGnpEFL was used as a plasmid for SeV reconstruction. As pSeV, pSeV(PM)EmGFP/TSdF, pSeV(PM)EmGFP-VAI74a/TSdF (loaded with VAI (180bp) 74a) and pSeV(PM)EmGFP-VAI74aL/TSdF (loaded with VAI (330bp) 74a) were used. EmGFP-VAI74a had a sequence set forth in SEQ ID NO: 32. EmGFP-VAI74aL had a sequence set forth in SEQ ID NO: 33.

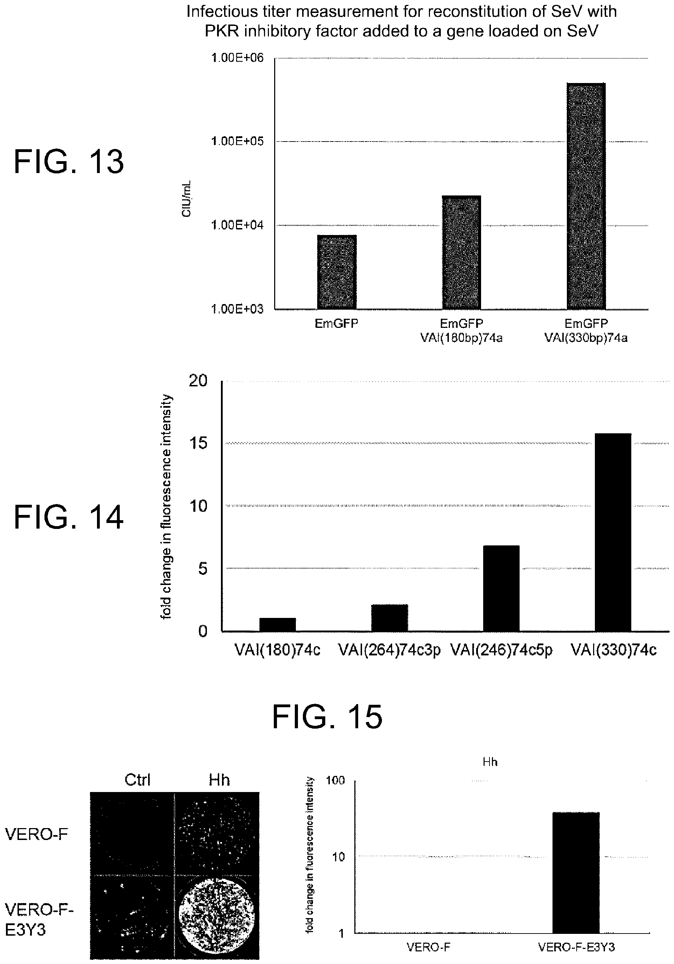

The results were as shown in Figures 12 and 13. As shown in Figure 12, the reconstruction efficiency of SeV loaded with VAI (180 bp) 74a was improved to 3 times, and the reconstruction efficiency of SeV loaded with VAI (330 bp) 74a was improved to 16 times, compared to the reconstruction efficiency of the control (Em-GFP). In addition, as shown in Figure 13, the infectious titer of the culture supernatant on the third day after the start of SeV reconstitution was 3 times higher for SeV loaded with VAI (180bp) 74a and 66 times higher for SeV loaded with VAI (330bp) 74a than the reconstitution efficiency of the control (EmGFP).

(14-2) Effect of addition, to VAI (180bp) 74c, of 5' sequence or 3' sequence of the relevant sequence

A cell population containing only LLC-MK2-F was used as cells for SeV reconstitution. CAGnpEFL was used as a plasmid for SeV reconstruction. pSeV+EmGFP/TSdF was used as pSeV. pT7-VAI (180 bp) 74c, pT7-VAI (264 bp) 74c3p, pT7-VAI (246 bp) 74c5p and pT7-VAI (330 bp) 74c were used as plasmids containing a PKR inhibitory factor. VAI (264 bp) 74c3p (SEQ ID NO: 34) is one obtained by adding an 84mer on the 3' side of VAI to the 3' side of VAI (180 bp) 74c. VAI (246 bp) 74c5p (SEQ ID NO: 35) is one obtained by adding an 86mer on the 5' side of VAI to the 5' side of VAI (180 bp) 74c.

Comparing the fluorescence intensities derived from EmGFP from cells on the third day after reconstitution to that in the presence of pT7-VAI (180 bp) 74c, as shown in Figure 14, the fluorescence intensity was 2.1 times higher in the presence of pT7-VAI (264 bp) 74c3p, 6.8 times higher in the presence of pT7-VAI (246 bp) 74c5p and 15.7 times higher in the presence of pT7-VAI (330 bp) 74c. This suggested that the sequence on the 5'-side of VAI contributes more strongly to an improvement in the reconstitution efficiency.

(15) Effect of EBER1 integration on virus proliferation

A cell population containing only LLC-MK2-F was used as cells for SeV reconstitution. CAGnpEFL was used as a plasmid for SeV reconstruction. pSeV+EmGFP/TSdF was used as pSeV. pT7-EBER (337 bp) was used as a plasmid containing a PKR inhibitory factor. This plasmid has sequences before and after VAI introduced before and after EBER1, respectively. Comparing the fluorescence intensities derived from EmGFP from cells on the third day after reconstitution, the fluorescence intensity was 3.8 times higher in the presence of EBER1 (337 bp) than that of the control.

(16) Effect of NS5A148 integration on virus proliferation

A cell population containing only LLC-MK2-F was used as cells for SeV reconstitution. CAGnpEFL was used as a plasmid for SeV reconstruction. pSeV+EmGFP/TSdF was used as pSeV. pCAGGS-NS5A148 was used as a plasmid containing a PKR inhibitor. This plasmid produces NS5A148 having an amino acid sequence set forth in SEQ ID NO: 16. Comparing the fluorescence intensities derived from EmGFP from cells on the third day after reconstitution, the fluorescence intensity was 7.3 times higher in the presence of NS5A148 than that of the control.

(17) Introduction of hammerhead ribozyme sequence

A cell population containing only Vero-F or cells containing only cloned Vero-F-E3Y3 was used as cells for SeV reconstitution. CAGnpEFL was used as a plasmid for SeV reconstruction. As pSeV, pSeV+EmGFP/TSdF, or pSeV+EmGFP/TSdF (Hh) having a hammerhead ribozyme sequence (SEQ ID NO: 36) under the sequence of a T7 promoter. Comparing the fluorescence intensities derived from EmGFP from cells on the third day after reconstitution, no EmGFP-positive cells were observed in Vero-F cells using pSeV + EmGFP/TSdF (control: Ctrl), whereas the EmGFP positive cells were observed in Vero-F-E3Y3 cells. As shown in Figure 15, the fluorescence intensity of EmGFP was 37.7 times higher in Vero-F-E3Y3 cells using pSeV+EmGFP/TSdF(Hh) than that in Vero-F cells.

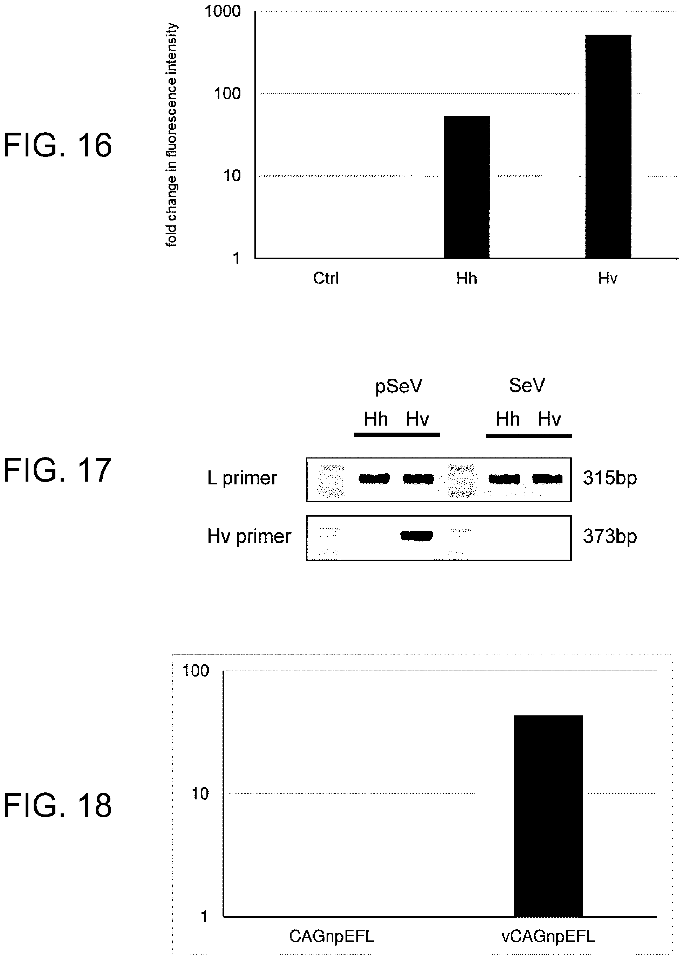

Another experiment was performed. A vector was constructed in which a hammerhead ribozyme (Hh-Rbz) sequence was positioned between the region encoding a T7 promoter and the region encoding a SeV genome of a plasmid that transcribes the SeV genome (pSeV/TSdF(Hh)). As Hh-Rbz, one having a sequence set forth in SEQ ID NO: 36 was used. A vector (pSeV/TSdF(Hv)) was constructed in which an HDV ribozyme (HDV-Rbz) sequence was further positioned directly downstream of the region encoding the SeV genome and VAI (330bp) 74c was further positioned downstream thereof. As HDV-Rbz, one having a sequence set forth in SEQ ID NO: 37 was used. A T7 terminator (SEQ ID NO: 38) was positioned downstream of VAI (330 bp) 74c. Thereby, when the SeV genome is transcribed, only the SeV genome portion is spliced out by the action of the hammerhead ribozyme and HDV ribozyme, and the other factor such as VAI is removed from the SeV genome. A cell population containing only LLC-MK2-F was used. CAGnpEFL was used as a plasmid for SeV reconstruction. pSeV+EmGFP/TSdF (control), pSeV+Em-GFP/TSdF (Hh) and pSeV+EmGFP/TSdF (Hv) were used as pSeV. Comparing the fluorescence intensities derived from EmGFP from cells on the third day after reconstitution, as shown in Figure 16, the fluorescence intensity was 52.5 times higher in the presence of pSeV+EmGFP/TSdF (Hh) than that of the control, and 506.7 times higher in the presence of pSeV+Em-GFP/TSdF (Hv) than that of the control.

It was investigated whether each of the SeV genomes obtained has VAI (330bp) 74c integrated therein by using the following primer set (L primer) that amplifies the L gene of the SeV genome and the following primer set (Hv primer) that amplifies VAI (330bp) 74c, respectively.

Forward primer for L gene amplification: TGGGTCATTCCCTGACCAGA (SEQ ID NO: 39)
Reverse primer for L gene amplification: CAGCTTCGATCGTTCTGCAC (SEQ ID NO: 40)
Forward primer for VAI amplification: ATCGAGCCTTATGACAGC (SEQ ID NO: 41)
Reverse primer for VAI amplification: GATACCCTTGCGAATTTATCCACC (SEQ ID NO: 42)

The SeV genome transcribed above was recovered from the Vero cells, and cDNA was synthesized. KOD One PCR Master Mix -Blue- (TOYOBO Co., Ltd.) was used as a PCR enzyme. As shown in Figure 17, the primer set (L primer) that amplifies the L gene amplified part of the L gene as expected in the plasmid (pSeV) loaded with the gene encoding SeV, and amplified part of the L gene as expected also in the SeV genome. In contrast, Hv primer did not amplify VAI (330 bp) 74c in the SeV genome. This suggests that after the SeV genome was transcribed from the plasmid, VAI (330 bp) 74c was cleaved from the SeV genome by HDV ribozyme and was lost. Even in that case, as shown in Figure 16, it is clear that the effect of improving the reconstruction efficiency of SeV was exerted.

(18) Introduction of VAI (330bp) 74c into plasmid for SeV reconstitution

In (17) above, the introduction of VAI (330 bp) 74c into a plasmid loaded with a gene encoding the SeV genome was described. In the present Example, VAI (330 bp) 74c was introduced into a plasmid expressing components of a SeV particle for SeV reconstitution. Specifically, a reconstitution experiment was performed as follows.

Plasmids for reconstitution such as pCAGGS and pEF1 that each express NP, P, L, T7 and F5R was additionally loaded with VAI (330 bp) 74c to obtain plasmids for reconstitution loaded with VAI (330 bp) 74c (designated as "pCAGGSv" and "pEFv", respectively). pCAGGSv-NPco, pCAGGSv-P4C(-)co, pEFv-Lco, pCAGGSv-T7mco and pCAGGSv-F5Rco, and combinations thereof are designated as "vCAGnpEFL". A cell population containing only LLC-MK2-F was used as cells for reconstitution. vCAGnpEFL was used as a plasmid for SeV reconstitution. CAG was used as a control. pSeV+EmGFP/TSdF was used as pSeV. Comparing the fluorescence intensities derived from EmGFP from cells on the third day after reconstitution, as shown in Figure 18, the fluorescence intensity was 43 time higher in the presence of vCAGnpEFL-mix than that of the control.

(19) Introduction of VAI (330bp) 74c into plasmid for SeV reconstitution

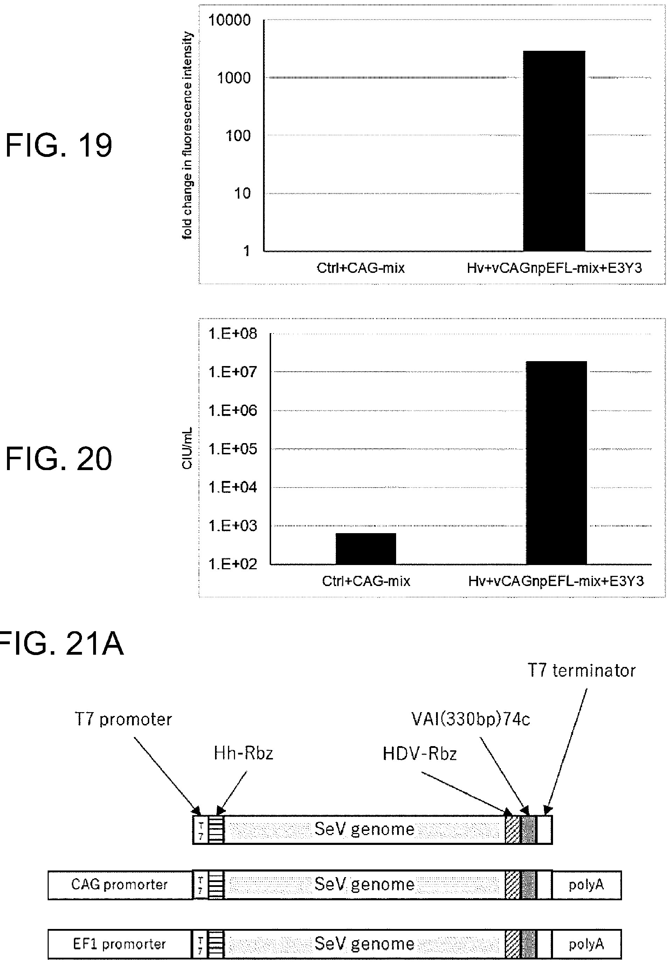

An experiment was conducted under conditions in which E3Y3 had been additionally introduced into the plasmid in (18) above. pSeV+EmGFP/TSdF (control) and pSeV+EmGFP/TSdF (Hv) were used as pSeV. Comparing the fluorescence intensities derived from EmGFP from cells on the third day after reconstitution, as shown in Figure 19, the fluorescence intensity was 2,836 time higher in the presence of Hv+vCAGnpEFL-mix+E3Y3 than that of the control. Comparing the infectious titers of the obtained culture supernatants, as shown in Figure 20, it was 29,925 times higher in the presence of Hv+vCAGnpEFL-mix+E3Y3 than that of the control. SeV reconstitution was possible even without use of F5R and E3Y3 during reconstitution.

(20) Regarding other vector constitutions

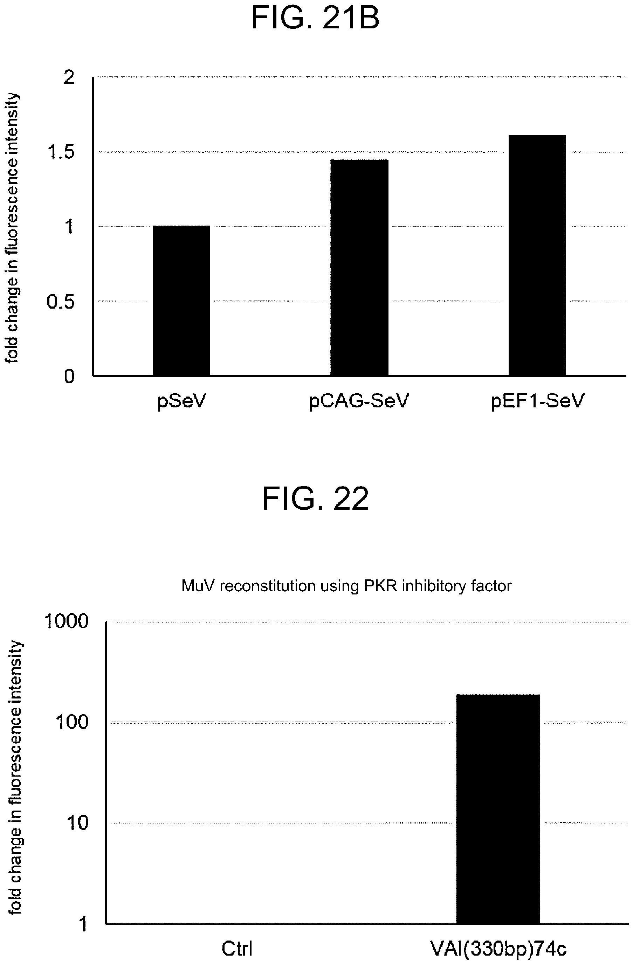

An SeV genome plasmid that was driven by a CAG promoter or an EF1 promoter in addition to one driven by the T7 promoter was constructed (see Figure 21A). An SeV genome plasmid having a CAG promoter is designated as pCAGGS-SeV, and an SeV genome plasmid having an EF1 promoter is designated as pEF1-SeV. A cell population containing only LLC-MK2-F was used as cells for reconstitution. CAGnpEFL was used as a plasmid for SeV reconstitution. pSeV+EmGFP/TSdF (Hv), pCAG-SeV+EmGFP/TSdF (Hv) or pEF1-SeV+Em-GFP/TSdF (Hv) was used as an SeV genome plasmid. Comparing the fluorescence intensities derived from Em-GFP from cells on the third day after reconstitution, as shown in Figure 21B, the fluorescence intensity was 1.44 time higher in the presence of pCAG-SeV than that of the control and 1.6 times higher in the presence of pEF1-SeV than that of the control.

(21) Reconstitution experiment of paramyxovirus other than SeV (MuV)

A cell population containing only LLC-MK2 was used as cells for reconstitution of mumps virus (MuV; Paramyxoviridae Rubulavirus). MuV-mix was used as a plasmid for MuV reconstitution. The MuV-mix contained the following plasmids: pCAGGS-MuV-N, pCAGGS-MuV-P, pCAGGS-MuV-L and pCAGGS-T7mco.

pMuV+EmGFP/mini was used as pMuV. pT7-VAI (330 bp) 74c was used as a plasmid containing a PKR inhibitor. The reference sequence for the MuV genome was the sequence registered as Accession: KY295913. MuV minigenome was constructed by removing the entire sequence between the leader sequence and trailer sequence from the above reference sequence.

In summary, the following plasmids were used in MuV reconstitution.

```
pMuV+EmGFP/mini
```

```
pCAGGS-MuV-N
pCAGGS-MuV-P
pCAGGS-MuV-L
pCAGGS-T7mco
```

Comparing the fluorescence intensities derived from EmGFP after MuV reconstitution from cells on the third day after reconstitution to that of the control, as shown in FIG. 22, the fluorescence intensity was 184 times higher in the presence of VAI (330 bp) 74c than that of the control.

(22) Reconstitution experiment of paramyxovirus other than SeV (MeV)

A cell population containing only LLC-MK2 was used as cells for reconstitution of measles virus (MeV; Paramyxoviridae Morbillivirus). MuV-mix was used as a plasmid for MeV reconstruction. The MeV-mix contained the following plasmids: pCAGGS-MeV-N, pCAGGS-MeV-P, pCAGGS-MeV-L and pCAGGS-T7mco. pMeV+EmGFP/mini was used as pMeV. pT7-VAI (330 bp) 74c was used as a plasmid containing a PKR inhibitor. The reference sequence for the MeV genome was the sequence registered as Accession: KY295921. MeV minigenome was constructed by removing the entire sequence between the leader sequence and trailer sequence from the above reference sequence.

In summary, the following plasmids were used in MeV reconstitution.

```
pMeV+EmGFP/mini
```

```
pCAGGS-MeV-N
pCAGGS-MeV-P
pCAGGS-MeV-L
pCAGGS-T7mco
pT7-VAI (330) 74c
```

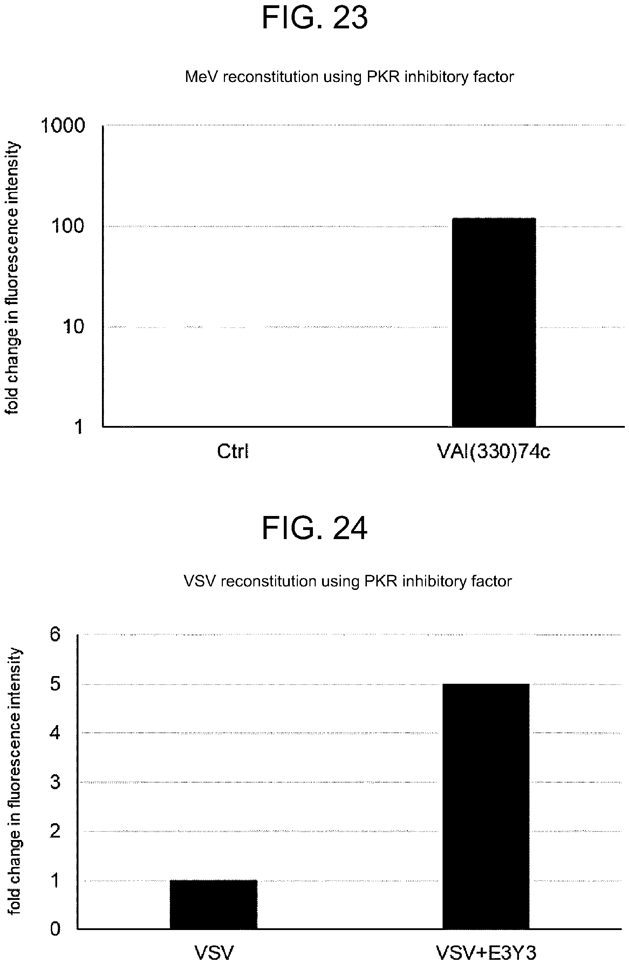

Comparing the fluorescence intensities derived from EmGFP after MeV reconstitution from cells on the third day after reconstitution to that of the control, as shown in FIG. 23, the reconstitution efficiency of MeV in the presence of VAI (330 bp) 74c was 120 times higher than that of the control.

(23) Reconstitution experiment of paramyxovirus other than SeV (VSV)

A cell population containing only LLC-MK2 was used as cells for reconstitution of vesicular stomatitis virus (VSV; Rhabdoviridae Vesiculovirus). VSV-mix (containing pCAGGS-VSV-N, pCAGGS-VSV-P, pCAGGS-VSV-L and pCAGGS-T7mco) was used for VSV reconstitution. pVSV-ΔG-GFP-2.6 (Kerafast, Inc.) was used as a VSV genome plasmid. pCAGGS-E3Y3 was used as a PKR inhibitor.

Comparing the fluorescence intensities derived from EmGFP from cells on the seventh day after reconstitution, as shown in Figure 24, the fluorescence intensity was 5 times higher when E3Y3 was used.

(24) Reconstitution of SeV, MeV and MuV using heterologous RNA polymerase

Hereinafter, a plasmid mixture for SeV reconstitution containing pCAGGS-NPco(SeV), pCAGGS-P4C(-)co(SeV), pEF1-Lco(SeV) and pCAGGS-T7mco is designated as "SeV-mix". A plasmid mixture for MeV reconstitution containing pCAGGS-MeV-N, pCAGGS-MeV-P, pCAGGS-MeV-L and pCAGGS-T7mco is designated as "MeV-mix." A plasmid mixture for MuV reconstitution containing pCAGGS-MuV-N, pCAGGS-MuV-P, pCAGGS-MuV-L and pCAGGS-T7mco is designated as "MuV-mix."

pMeV+EmGFP/mini having a MeV minigenome loaded with EmGFP which does not express MeV constituent proteins (N, P, M, F, H and L) was used as a MeV genome plasmid. pMuV+EmGFP/mini having a MuV minigenome loaded with EmGFP which does not express MuV constituent proteins (N, P, M, F, SH, HN and L) was used as a MuV genome plasmid. pSeV+EmGFP/TSdF (Hv) was used as an SeV genome plasmid. A cell population containing only LLC-MK2-F was used as cells for reconstituting these.

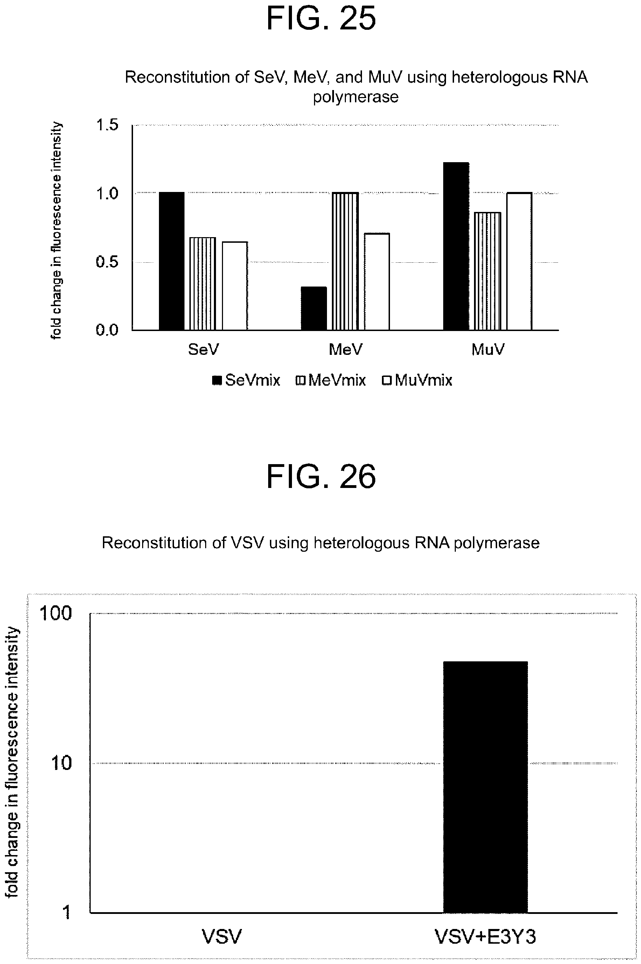

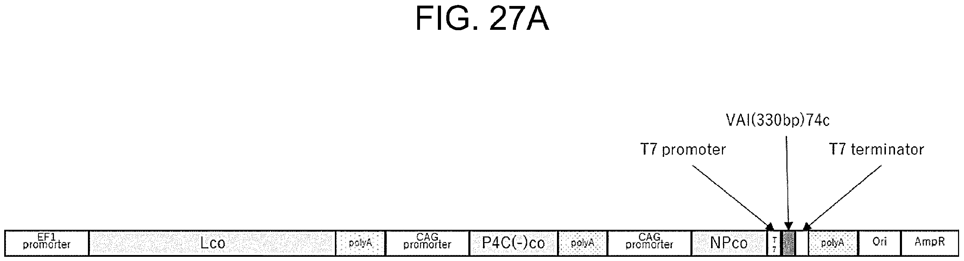

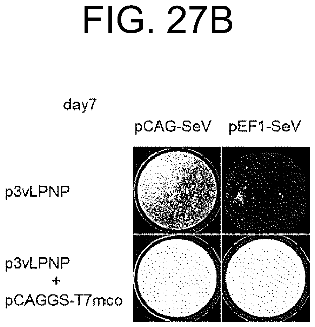

Comparing the fluorescence intensities derived from EmGFP from cells on the third day after reconstitution, as shown in Figure 25, the SeV genome was reconstituted not only when using the SeV-mix but also when using each of the heterologous polymerases of the MeV-mix and the MuV-mix. Similarly, the MeV genome and the MuV genome were also reconstituted with the heterologous polymerases. Comparing the fluorescence intensities, setting the fluorescence intensity when the SeV genome was reconstructed using SeV-mix as 1, the fluorescence intensity was 0.68 when using the MeV-mix and 0.65 when using the MuV-mix (see Figure 27). Comparing the fluorescence intensities, setting the fluorescence intensity when the MeV genome was reconstructed using MeV-mix as 1, the fluorescence intensity was 0.31 when using the SeV-mix and 0.71 when using the MuV-mix (see Figure 25). Comparing the fluorescence intensities, setting the fluorescence intensity when the MuV genome was reconstructed using MuV-mix as 1, the fluorescence intensity was 1.22 when using the SeV-mix and 0.86 when using the MEV-mix (see Figure 25).

(25) Construction of VSV using heterologous RNA polymerase

A cell population containing only LLC-MK2-F was used as cells for VSV reconstitution. SeV-mix was used for VSV reconstitution. pVSV-ΔG-GFP-2.6 (Kerafast, Inc.) was used as a VSV genome plasmid. pCAGGS-E3Y3 was used as a PKR inhibitor.

Comparing the fluorescence intensities derived from EGFP from cells on the third day after reconstitution, as shown in Figure 26, the VSV genome was confirmed to be reconstituted with the SeV-mix, and when E3Y3 was additionally introduced into the cells, the fluorescence intensity was 47.5 times higher than that in the absence of E3Y3.

Furthermore, a plasmid (p3vLPNP) that expresses NP, P and L from the one plasmid was constructed (see Figure 27A). pSeV avoided the use of a T7 polymerase by causing the pSeV to be driven by a CAG promoter (pCAG-SeV) or an EF1 promoter (pEF1-SeV). SeV was reconstituted with a cell population containing only LLC-MK2 in the same manner as above. The results were as shown in Figure 27B. As shown in Figure 27B, 7 days after reconstitution, the fluorescence of EmGFP was observed for both pCAG-SeV and pEF1-SeV, and it was confirmed that the SeV had been reconstituted. When the T7 polymerase was additionally supplied by the pCAGGS-T7mco plasmid, the efficiency of reconstitution was greatly improved.

Description of sequence listing

SEQ ID NO: 1: an example of an EF1α promoter sequence
SEQ ID NO: 2: an example of a CAG promoter sequence
SEQ ID NO: 3: an example of a sequence of a gene encoding EmGFP
SEQ ID NO: 4: sequence of EBER of an EB virus
SEQ ID NO: 5: a sequence of TAR of HIV
SEQ ID NO: 6: 2A$^{pro}$ of poliovirus
SEQ ID NO: 7: E3L of vaccinia virus
SEQ ID NO: 8: σ3 of reovirus
SEQ ID NO: 9: human p58$^{IPK}$
SEQ ID NO: 10: K3L of vaccinia virus
SEQ ID NO: 11: Tat of HIV
SEQ ID NO: 12: ICP34.5 of herpes simplex virus
SEQ ID NO: 13: nc866
SEQ ID NO: 14: a long version of nc866
SEQ ID NO: 15: NS5A
SEQ ID NO: 16: NS5A (1-148)

SEQ ID NO: 17: VAI (180mer)
SEQ ID NO: 18: VAI (c.74U>V)
SEQ ID NO: 19: VAI (330mer)
SEQ ID NO: 20: VAI (330mer; c.74U>V)
SEQ ID NO: 21: VAI-VAII
SEQ ID NO: 22: VAI (180mer: c.74U>M)
SEQ ID NO: 23: VAI (330mer: c.74U>M)
SEQ ID NO: 24: VAI (330mer, c.191C>D)
SEQ ID NO: 25: VAI (330mer, c.74U>V, c.191C>D)
SEQ ID NO: 26: VAI (330mer, c.74U>M, c.191C>G)
SEQ ID NO: 27: C-terminal 106 amino acids of C protein of a Sendai virus
SEQ ID NO: 28: NS1 of influenza virus
SEQ ID NO: 29: Us11 of herpes simplex virus
SEQ ID NO: 30: a fusion protein E3K3
SEQ ID NO: 31: a fusion protein E3Y3
SEQ ID NO: 32: EmGFP-VAI (74 U>A)
SEQ ID NO: 33: EmGFP-VAI74aL (74 U>A)
SEQ ID NO: 34: VAI (264bp) 74c3p
SEQ ID NO: 35: VAI (246bp) 74c5p
SEQ ID NO: 36: an example of a sequence of Hh-Rbz
SEQ ID NO: 37: an example of a sequence of HDV-Rbz
SEQ ID NO: 38: an example of a T7 terminator sequence
SEQ ID NO: 39: a forward primer for L gene amplification
SEQ ID NO: 40: a reverse primer for L gene amplification
SEQ ID NO: 41: a forward primer for VAI amplification
SEQ ID NO: 42: a reverse primer for VAI amplification

## Claims

1. A method of producing a negative-strand RNA virus or virus vector, the method comprising:

   expressing a protein kinase R (PKR) inhibitory factor from a gene encoding the PKR inhibitory factor operably linked to a regulatory sequence and supplying it to a packaging cell;
   causing the packaging cell to express a genomic RNA of the negative-strand RNA virus or virus vector to form the negative-strand RNA virus or virus vector in the presence of the PKR inhibitory factor; and
   recovering the formed negative-strand RNA virus or virus vector;
   wherein:

   the PKR inhibitory factor is a PKR inhibitory viral factor, or nc886 or p58$^{IPK}$; and
   the relationship between the virus or virus vector and the PKR inhibitory factor is heterologous, and/or the relationship between the regulatory sequence and the PKR inhibitory factor is heterologous.

2. The method according to claim 1, wherein the negative-strand RNA virus or virus vector is a Sendai virus vector.

3. The method according to claim 1 or 2, wherein the PKR inhibitory factor is one or more selected from the group consisting of VAI RNA of adenovirus, EBER of EB virus, TAR of HIV virus, 2Apro of poliovirus, E3L of vaccinia virus, δ3 of reovirus, NS1 of influenza virus, human p58IPK, NS5A of hepatitis C virus, K3L of vaccinia virus, Tat of HIV virus, human nc886, Us11 of herpes simplex virus and ICP34.5 of herpes simplex virus, and orthologs thereof.

4. The method according to any one of claims 1 to 3, wherein the negative-strand RNA virus or virus vector is produced in the absence of a helper virus.

5. The method according to any one of claims 1 to 4, wherein the genomic RNA further has the gene encoding the PKR inhibitory factor operably linked to the regulatory sequence.

6. The method according to any one of claims 1 to 5, wherein the packaging cell has a genomic DNA having the gene encoding the PKR inhibitory factor operably linked to the regulatory sequence.

7. The method according to any one of claims 1 to 6, wherein the packaging cell is a Vero cell or an LLC-MK2 cell.

8. The method according to any one of claims 1 to 7, wherein the packaging cell is a cell population consisting of Vero cells or a cell population consisting of LLC-MK2 cells, which does not comprise other cells.

9. An RNA genome of a negative-strand RNA virus or virus vector, comprising expressibly a gene encoding any one or more of PKR inhibitory factors.

10. A negative-strand RNA virus or virus vector comprising the RNA genome according to claim 9.

11. The negative-strand RNA virus or virus vector according to claim 10, further comprising a gene of interest.

12. A composition comprising the negative-strand RNA virus or virus vector according to claim 10 or 11.

13. A DNA encoding the RNA genome according to claim 9.

14. A gene expression vector comprising the DNA according to claim 13 operably linked to a regulatory sequence.

# FIG. 1A

Structure of VAI RNA

In order to remove the BanHI site (ggatcc) without disrupting the apical stem structure of VAI, a mutation can be introduced at the 74th base.

# FIG. 1B

VAI is underlined.

VAI(180bp) wt

cuguaagcGGGCACUCUUCCGUGGUCUGGUGGAUAAAUUCGCAAGGGUAUCAUGGCGGACGACCGGGGUUCGAACCCCGGAUCCGGCCGUCCGCCGUGAUCCAUGCGGUUACCGCCCGCGUGUCGAACCCAGGUGUGCGACGUCAGACAACGGGGGAGCGCUCCUUUUggcuuccuucca

VAI(180bp) 74a

cuguaagcGGGCACUCUUCCGUGGUCUGGUGGAUAAAUUCGCAAGGGUAUCAUGGCGGACGACCGGGGUUCGAACCCCGGAaCCGGCCGUCCGCCGUGAUCCAUGCGGUUACCGCCCGCGUGUCGAACCCAGGUGUGCGACGUCAGACAACGGGGGAGCGCUCCUUUUggcuuccuucca

VAI(180bp) 74c

cuguaagcGGGCACUCUUCCGUGGUCUGGUGGAUAAAUUCGCAAGGGUAUCAUGGCGGACGACCGGGGUUCGAACCCCGGAcCCGGCCGUCCGCCGUGAUCCAUGCGGUUACCGCCCGCGUGUCGAACCCAGGUGUGCGACGUCAGACAACGGGGGAGCGCUCCUUUUggcuuccuucca

VAI(330bp) 74a

gucgggacgcucuggccggugaggcgugcgcagucguugacgcucuagaccgugcaaaaggagagccuguaagcGGGCACUCUUCCGUGGUCUGGUGGAUAAAUUCGCAAGGGUAUCAUGGCGGACGACCGGGGUUCGAACCCCGGAaCCGGCCGUCCGCCGUGAUCCAUGCGGUUACCGCCCGCGUGUCGAACCCAGGUGUGCGACGUCAGACAACGGGGGAGCGCUCCUUUUggcuuccuuccaggcgcggcggcugcugcgGuagcuuuuuuggccacuggccgcgcgcggcguaagcgguuaggcuggaaagcgaaagcauuaagu

In order to remove the NheI site (gctagc), a mutation can also be introduced at 191st base.

VAI-VAII(478bp) wt

gcgcagucguugacgcucuagaccgugcaaaaggagagccuguaagcGGGCACUCUUCCGUGGUCUGGUGGAUAAAUUCGCAAGGGUAUCAUGGCGGACGACCGGGGUUCGAACCCCGGAUCCGGCCGUCCGCCGUGAUCCAUGCGGUUACCGCCCGCGUGUCGAACCCAGGUGUGCGACGUCAGACAACGGGGGAGCGCUCCUUUUggcuuccuuccaggcgcggcggcugcugcgcuagcuuuuuuggccacuggccgcgcgcggcguaagcgguuaggcuggaaagcgaaagcauuaaguGGCUCGCUCCCUGUAGCCGGAGGGUUAUUUUCCAAGGGUUGAGUCGCAGGACCCCCGGUUCGAGUCUCGGGCCGGCCGGACUGCGGCGAACGGGGGUUUGCCUCCCCGUCAUGCAAGACCCCGCUUGCAAAUUCCUCCGGAAACAGGGACGAGCCCCUUUUUUgcuuuuccaga

## SeV reconstitution using VAI

**FIG. 2**

## Infectious titer measurement for SeV reconstitution using VAI

**FIG. 3**

FIG. 4

SeV reconstitution using nc886

FIG. 5

SeV reconstitution using PKR inhibitory factor

FIG. 6

Infectious titer measurement for SeV reconstitution using PKR inhibitory factor

# FIG. 7

SeV reconstitution using fusion sequence of PKR inhibitory factor

# FIG. 8

# FIG. 9

# FIG. 10

SeV reconstitution using combination of PKR inhibitory factors

# FIG. 11

Infectious titer measurement for SeV reconstitution using
combination of PKR inhibitory factors

# FIG. 12

Reconstitution of SeV with PKR inhibitory factor
added to a gene loaded on SeV

Infectious titer measurement for reconstitution of SeV with
PKR inhibitory factor added to a gene loaded on SeV

FIG. 13

FIG. 14

FIG. 15

FIG. 16

FIG. 17

FIG. 18

FIG. 19

FIG. 20

FIG. 21A

## FIG. 21B

## FIG. 22

MuV reconstitution using PKR inhibitory factor

# FIG. 23

MeV reconstitution using PKR inhibitory factor

# FIG. 24

VSV reconstitution using PKR inhibitory factor

# FIG. 25

Reconstitution of SeV, MeV, and MuV using heterologous RNA polymerase

■ SeVmix   ⊞ MeVmix   ☐ MuVmix

# FIG. 26

Reconstitution of VSV using heterologous RNA polymerase

FIG. 27A

# FIG. 27B

## INTERNATIONAL SEARCH REPORT

| International application No. |
|---|
| **PCT/JP2022/025983** |

**A. CLASSIFICATION OF SUBJECT MATTER**

*C12N 15/86*(2006.01)i; *C12N 5/071*(2010.01)i; *C12N 7/01*(2006.01)i; *C12N 15/38*(2006.01)i; *C12N 15/39*(2006.01)i; *C12N 15/44*(2006.01)i; *C12N 15/49*(2006.01)i
FI:   C12N15/86 Z; C12N15/44; C12N15/39; C12N15/49; C12N15/38; C12N5/071; C12N7/01 ZNA

According to International Patent Classification (IPC) or to both national classification and IPC

**B. FIELDS SEARCHED**

Minimum documentation searched (classification system followed by classification symbols)

   C12N15/86; C12N5/071; C12N7/01; C12N15/38; C12N15/39; C12N15/44; C12N15/49

Documentation searched other than minimum documentation to the extent that such documents are included in the fields searched

   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)

   JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | KR 10-2020-0069259 A (NATIONAL CANCER CENTER) 16 June 2020 (2020-06-16) claims, examples | 1-14 |
| Y | | 1-14 |
| Y | JP 2015-534826 A (BIONTECH AG) 07 December 2015 (2015-12-07) claims, examples, paragraphs [0001]-[0282] | 1-14 |
| Y | JP 2020-534810 A (BIONTECH RNA PHARMACEUTICALS GMBH) 03 December 2020 (2020-12-03) claims, examples, paragraphs [0001]-[0298] | 1-14 |
| Y | JP 2001-514519 A (VIROGENETICS CORPORATION et al.) 11 September 2001 (2001-09-11) claims, pp. 4-21 | 1-14 |
| Y | JP 2001-514518 A (VIROGENETICS CORPORATION) 11 September 2001 (2001-09-11) claims, p. 5 to p. 26, line 12 | 1-14 |

✓ Further documents are listed in the continuation of Box C.      ✓ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 September 2022** | **20 September 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/025983**

**C.    DOCUMENTS CONSIDERED TO BE RELEVANT**

| Category* | Citation of document, with indication, where appropriate, of the relevant passages | Relevant to claim No. |
|---|---|---|
| Y | JP 2005-534326 A (BAVARIAN NORDIC A/S) 17 November 2005 (2005-11-17) claims, examples | 1-14 |
| Y | WO 2005/071092 A1 (DNAVEC RESEARCH INC) 04 August 2005 (2005-08-04) claims, examples, paragraphs [0001]-[0079] | 1-14 |

Form PCT/ISA/210 (second sheet) (January 2015)

## INTERNATIONAL SEARCH REPORT

| International application No. |
|---|
| **PCT/JP2022/025983** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/025983**

| Patent document cited in search report | | | Publication date (day/month/year) | Patent family member(s) | | | Publication date (day/month/year) |
|---|---|---|---|---|---|---|---|
| KR | 10-2020-0069259 | A | 16 June 2020 | US | 2022/0025338 | A1 | |
| | | | | claims, examples | | | |
| JP | 2015-534826 | A | 07 December 2015 | US | 2019/0269793 | A1 | |
| | | | | claims, examples, paragraphs [0001]-[0335] | | | |
| | | | | EP | 2917350 | A1 | |
| JP | 2020-534810 | A | 03 December 2020 | US | 2020/0362313 | A1 | |
| | | | | claims, examples, paragraphs [0001]-[0396] | | | |
| | | | | EP | 3681901 | A1 | |
| JP | 2001-514519 | A | 11 September 2001 | US | 6004777 | A | |
| | | | | claims, columns 1-10 | | | |
| | | | | EP | 968295 | A1 | |
| JP | 2001-514518 | A | 11 September 2001 | US | 5990091 | A | |
| | | | | claims, columns 1-13 | | | |
| | | | | EP | 970226 | A1 | |
| JP | 2005-534326 | A | 17 November 2005 | US | 2006/0039928 | A1 | |
| | | | | claims, examples | | | |
| | | | | EP | 1529113 | A1 | |
| WO | 2005/071092 | A1 | 04 August 2005 | US | 2007/0161110 | A1 | |
| | | | | claims, examples, paragraphs [0001]-[0171] | | | |
| | | | | EP | 1717317 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005071092 A
- WO 98040500 A
- WO 95011983 A
- WO 2005071085 A
- WO 2003025570 A

**Non-patent literature cited in the description**

- **T. SHORS et al.** *Virology,* 1997, vol. 239, 269-276
- **M. K. KOBAYASHI et al.** *Virology,* 2000, vol. 276, 424-434
- **T. TAGUCHI et al.** *J. Gen. Virol.,* 2004, vol. 85, 959-969
- *Medical Journal of Kobe University,* 2003, vol. 64 (1/2), 7-15
- *J. General Virology,* 1997, vol. 78, 2813-2820
- **BEATY, SM. et al.** *mSphere,* 2017, vol. 2, e00376-16